# EUROPEAN PATENT APPLICATION

(11) **EP 4 487 764 A1**
(43) Date of publication of application: **08.01.2025**
(21) Application number: 23763429.0
(22) Date of filing: 28.02.2023
(51) Int. Cl.: A61B 5/02, G01B 7/16

(54) **PULSE WAVE SENSOR**

(30) Priority: 04.03.2022 JP 2022033658; 01.02.2023 JP 2023014131
(71) Applicant: Minebea Mitsumi Inc., Kitasaku-gun, Nagano 3890293 (JP)
(72) Inventor: KITAZUME, Makoto, Tama-shi, Tokyo 206-8567 (JP); INAMOTO, Shigenori, Tama-shi, Tokyo 206-8567 (JP); ASAKAWA, Toshiaki, Kitasaku-gun, Nagano 389-0293 (JP); KITAMURA, Atsushi, Kitasaku-gun, Nagano 389-0293 (JP)
(74) Representative: Zabel, Julia Elisabeth
(86) International application number: PCT/JP2023/007261
(87) International publication number: WO 2023/167172

(57) **Abstract**

This pulse wave sensor comprises: a strain-generating body having a circular opening; a resin layer covering one surface of the strain-generating body; and a strain gauge which is disposed on the other surface opposite to the one surface of the strain-generating body and has a Cr mixed phase film as a resistor, wherein 0.059≤t≤0.124 when the material of the strain-generating body is SUS and d=32, 0.046≤t≤0.099 when the material is SUS and d=22, 0.030≤t≤0.067 when the material is SUS and d=13, 0.026≤t≤0.034 when the material is SUS and d=7, 0.084≤t≤0.166 when the material is copper and d=32, 0.066≤t≤0.132 when the material is copper and d=22, 0.044≤t≤0.088 when the material is copper and d=13, 0.032≤t≤0.050 when the material is copper and d=7, 0.097≤t≤0.212 when the material is aluminum and d=32, 0.079≤t≤0.168 when the material is aluminum and d=22, 0.050≤t≤0.107 when the material is aluminum and d=13, and 0.038≤t≤0.063 when the material is aluminum and d=7, where d [mm] is the diameter of the circular opening and t [mm] is the thickness of the strain-generating body. Pulse wave detection is based on the change in the resistance value of the resistor accompanying the deformation of the strain-generating body.

## Description

### TECHNICAL FIELD

The present invention relates to a pulse wave sensor.

### BACKGROUND ART

A pulse wave sensor for detecting arterial pulses generated when the heart pumps blood is known. An example of the pulse wave sensor is a pulse wave sensor provided with a pressure receiving plate as a strain generating body supported in a deflective manner by an action of an external force and a piezoelectric conversion element for converting the deflection of the pressure receiving plate into an electric signal. The pulse wave sensor has a dome-like shape in which a deflective region of the pressure receiving plate becomes a convex curved surface outward, and a pressure detecting element is provided on the inner surface of the top of the pressure receiving plate as the piezoelectric conversion element (for example, Patent Document 1).

### CITATION LIST

### PATENT DOCUMENT

[Patent Document 1] Japanese Unexamined Patent Application Publication No. 2002-78689

### SUMMARY OF THE INVENTION

### TECHNICAL PROBLEM

Since a pulse wave sensor needs to detect a minute signal, a thin strain generating body is used to ensure the required sensitivity, but on the other hand, durability is also required. Therefore, the pulse wave sensor that is compatible with rigidity and durability is required. However, compatibility between sensitivity and rigidity has not been sufficiently investigated in conventional pulse wave sensors.

An object of the present invention is to provide a pulse wave sensor having both sensitivity and rigidity in view of the above.

### SOLUTION TO THE PROBLEM

A pulse wave sensor includes a strain generating body with a circular opening, a resin layer covering one surface of the strain generating body, and a strain gauge provided on the other surface of the strain generating body positioned on a side opposite to the one surface, the strain gauge including a Cr mixed phase film as a resistor. Where a diameter of the circular opening is d [mm] and a thickness of the strain generating body is t [mm], when a material of the strain generating body is SUS and d is 32, a required range of t is 0.059≤t≤0.124, when the material is SUS and d is 22, a required range of t is 0.046≤t≤0.099, when the material is SUS and d is 13, a required range of t is 0.030≤t≤0.067, when the material is SUS and d is 7, a required range of t is 0.026≤t≤0.034, when the material is copper and d is 32, a required range of t is 0.084≤t≤0.166, when the material is copper and d is 22, a required range of t is 0.066≤t≤0.132, when the material is copper and d is 13, a required range of t is 0.044≤t≤0.088, when the material is copper and d is 7, a required range of t is 0.032≤t≤0.050, when the material is aluminum and d is 32, a required range of t is 0.097≤t≤0.212, when the material is aluminum and d is 22, a required range of t is 0.079≤t≤0.168, when the material is aluminum and d is 13, a required range of t is 0.050<t<0.107, when the material is aluminum and d is 7, a required range of t is 0.038<t<0.063. The pulse wave sensor is configured to detect a pulse wave based on a change in a resistance value of the resistor in response to a deformation of the strain generating body.

### ADVANTAGEOUS EFFECTS OF THE INVENTION

According to the disclosed technique, the present invention can provide a pulse wave sensor having both sensitivity and rigidity.

### BRIEF DESCRIPTION OF THE DRAWINGS

[FIG. 1] FIG. 1 is a perspective view illustrating a pulse wave sensor according to a first embodiment.
[FIG. 2] FIG. 2 is a plan view illustrating the pulse wave sensor according to the first embodiment.
[FIG. 3] FIG. 3 is a cross-sectional view illustrating the pulse wave sensor of the first embodiment and illustrates a cross-section along the A-A line in FIG. 2.
[FIG. 4] FIG. 4 is a graph (No. 1) of the result of an experiment using SUS as a material for a strain generating body.
[FIG. 5] FIG. 5 is a graph (No. 2) of the result of an experiment using SUS as a material for a strain generating body.
[FIG. 6] FIG. 6 is a graph (No. 3) of the result of an experiment using SUS as a material for a strain generating body.
[FIG. 7] FIG. 7 is a graph (No. 4) of the result of an experiment using SUS as a material for a strain generating body.
[FIG. 8] FIG. 8 is a graph (No. 1) of the result of an experiment using copper as a material for a strain generating body.
[FIG. 9] FIG. 9 is a graph (No. 2) of the result of an experiment using copper as a material for a strain generating body.
[FIG. 10] FIG. 10 is a graph (No. 3) of the result of an experiment using copper as a material for a strain generating body.
[FIG. 11] FIG. 11 is a graph (No. 4) of the result of an experiment using copper as a material for a strain generating body.
[FIG. 12] FIG. 12 is a graph (No. 1) of the result of an experiment using aluminum as a material for a strain generating body.
[FIG. 13] FIG. 13 is a graph (No. 2) of the result of an experiment using aluminum as a material for a strain generating body.
[FIG. 14] FIG. 14 is a graph (No. 3) of the result of an experiment using aluminum as a material for a strain generating body.
[FIG. 15] FIG. 15 is a graph (No. 4) of the result of an experiment using aluminum as a material for a strain generating body.
[FIG. 16] FIG. 16 is a plan view illustrating a strain gauge according to the first embodiment.
[FIG. 17] FIG. 17 is a cross-sectional view illustrating the strain gauge according to the first embodiment.
[FIG. 18] FIG. 18 is a cross-sectional view (No. 1) illustrating a pulse wave sensor according to a modified example of the first embodiment.
[FIG. 19] FIG. 19 is a cross-sectional view (No. 2) illustrating a pulse wave sensor according to a modified example of the first embodiment.
[FIG. 20] FIG. 20 is a cross-sectional view (No. 3) illustrating a pulse wave sensor according to a modified example of the first embodiment.
[FIG. 21] FIG. 21 includes a plan view and a cross-sectional view illustrating an example of a detecting element included in a strain gauge according to a second embodiment.
[FIG. 22] FIG. 22 includes a perspective view, a plan view, and a cross-sectional view illustrating an example of a detecting element included in a strain gauge according to a third embodiment.
[FIG. 23] FIG. 23 includes a perspective view, a plan view, and a cross-sectional view illustrating another example of a detecting element included in the strain gauge according to the third embodiment.
[FIG. 24] FIG. 24 includes a perspective view, a plan view, and a cross-sectional view illustrating yet another example of a detecting element included in the strain gauge according to the third embodiment.

### DETAILED DESCRIPTION OF THE INVENTION

Hereinafter, the embodiments for carrying out the invention will be described with reference to the drawings. In each drawing, the same component parts are denoted by the same reference numerals, and duplicate descriptions may be omitted.

### <First Embodiment>

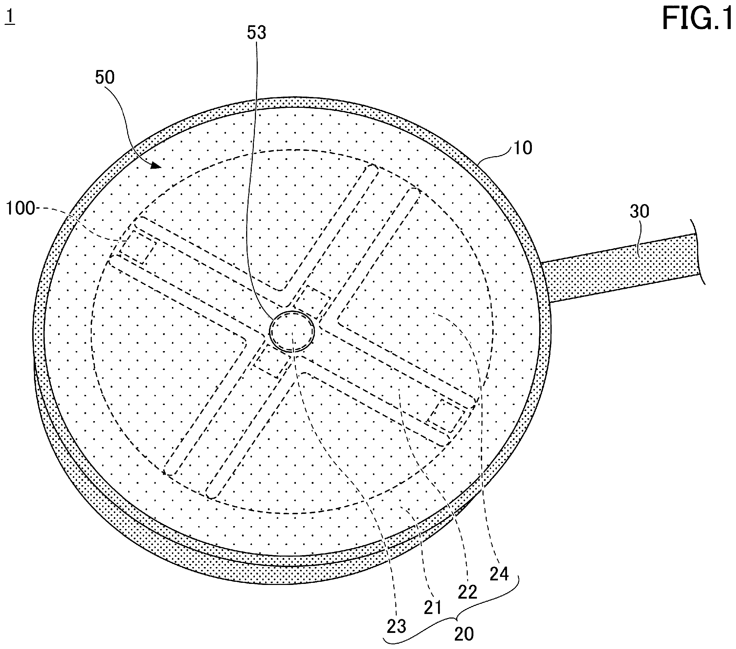

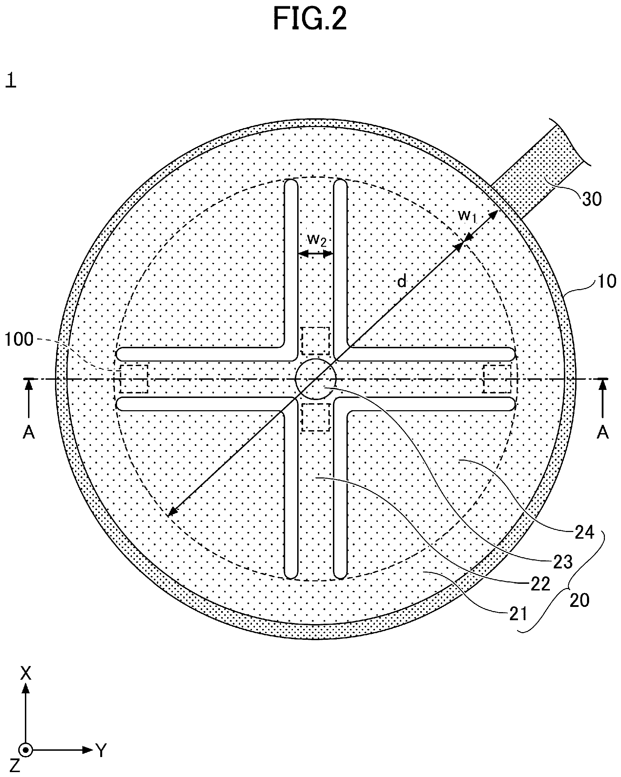

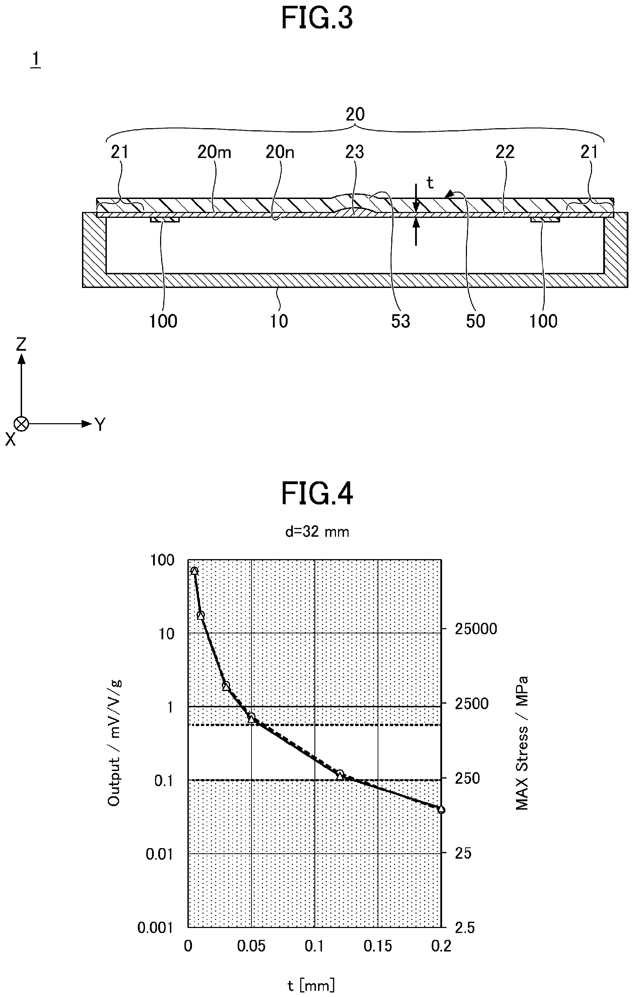

FIG. 1 is a perspective view illustrating a pulse wave sensor according to the first embodiment. FIG. 2 is a plan view illustrating the pulse wave sensor according to the first embodiment. FIG. 3 is a cross-sectional view illustrating the pulse wave sensor according to the first embodiment and illustrates a cross-section along the A-A line in FIG. 2. Although FIG. 2 does not illustrate a resin layer 50, a resin layer 50 also exists in FIG. 2 at a position corresponding to the position at which it is illustrated in FIG. 1.

Referring to FIGS. 1 to 3, the pulse wave sensor 1 includes a housing 10, a strain generating body 20, a wire rod 30, a resin layer 50, and a strain gauge 100.

The strain generating body 20 includes a base portion 21, a beam portion 22, a load portion 23, and an extension portion 24. The strain generating body 20 has a flat plate shape, and each component is integrally formed by, for example, a pressing process and the like. The strain generating body 20 is, for example, four-fold symmetrical in a plan view. The thickness t of the strain generating body 20 excluding the load portion 23 is constant. A suitable range of the thickness t will be described later.

In the present embodiment, for convenience, in the pulse wave sensor 1, the side where the load portion 23 of the strain generating body 20 is provided is the upper side or one side, and the side where the load portion 23 is not provided is the lower side or the other side. In addition, a surface of each part on a side where the load portion 23 is provided is one surface or the upper surface, and a surface of each part on a side where the load portion 23 is not provided is the other surface or the lower surface. However, the pulse wave sensor 1 can be used in an inverted state or arranged at any angle. The plan view refers to the object viewed in the direction normal to the upper surface of the strain generating body 20, and the planar shape refers to the shape of the object viewed in the direction normal to the upper surface of the strain generating body 20.

In the pulse wave sensor 1, the housing 10 holds the strain generating body 20. The housing 10 has a hollow cylindrical shape, and the lower surface side is closed and the upper surface side is opened. The housing 10 can be formed of, for example, metal, resin, or the like. In order to close the opening on the upper surface side of the housing 10, a substantially disk-shaped strain generating body 20 is fixed by an adhesive or the like.

In the strain generating body 20, the base portion 21 is a circular frame-shaped (ring-shaped) region outside the circular dashed line illustrated in FIGS. 1 and 2. The region inside the circular dashed line may be called a circular opening. That is, the base portion 21 of the strain generating body 20 has the circular opening. The width w₁ of the base portion 21 is, for example, in a range from 1 mm to 5 mm. A suitable range of the inner diameter d (that is, the diameter of the circular opening) of the base portion 21 will be described later.

The beam portion 22 is provided so as to bridge the inside of the base portion 21. The beam portion 22 has, for example, two beams that cross each other in a cross shape in a plan view, and the region where the two beams cross includes a center of the circular opening. In the example of FIG. 2, one beam that forms a cross has its longitudinal direction in the X-direction, and the other beam that forms a cross has its longitudinal direction in the Y direction, and they are orthogonal to each other. Each of the two orthogonal beams is disposed inward relative to the inner diameter d (diameter of the circular opening) of the base portion 21 and is preferably as long as possible. That is, the length of each beam is preferably approximately equal to the diameter of the circular opening. In each beam constituting the beam portion 22, the width w₂ other than the region where the two beams cross is constant, for example, in a range from 1 mm to 5 mm. The width w₂ is not necessarily constant. However, it is preferable in that the strain can be detected linearly by making the width w₂ constant.

The load portion 23 is provided on the beam portion 22. The load portion 23 is provided, for example, on a region where two beams constituting the beam portion 22 cross. The load portion 23 projects from the upper surface of the beam portion 22. The amount of projection of the load portion 23 relative to the upper surface of the beam portion 22 is, for example, about 0.1 mm. The beam portion 22 is flexible and elastically deformed when a load is applied to the load portion 23.

The four extension portions 24 are sectorial parts extending in the direction of the beam portion 22 from the inside of the base portion 21 in a plan view. A gap of about 1 mm is provided between each extension portion 24 and the beam portion 22. Since the extension portion 24 does not contribute to the sensing of the pulse wave sensor 1, the extension portion 24 does not need to be provided.

The wire rod 30 is a cable for inputting/outputting electric signals between the pulse wave sensor 1 and the outside. The wire rod 30 may be a shield cable, a flexible board, or the like.

The resin layer 50 covers one surface of the strain generating body 20. In the present embodiment, the one surface is an upper surface 20m. The resin layer 50 may cover the entirety of the upper surface 20m of the strain generating body 20, or may cover a part of the upper surface 20m. The resin layer 50 is also formed over the gaps between the beam portion 22 and the extension portions 24. Thus, the gaps between the beam portion 22 and the extension portions 24 are not exposed to the outside of the pulse wave sensor 1. The resin layer 50 may enter the gaps between the beam portion 22 and the extension portions 24 and fill a part or the whole of each of the gaps between the beam portion 22 and the extension portions 24.

As the resin layer 50, it is preferable to use a resin material having an elastic modulus of 10 GPa or lower. Examples of such a resin material include an epoxy resin, a silicone resin, and the like. When a resin material having an elastic modulus of 10 GPa or lower is used, the resin material constituting the resin layer 50 does not inhibit elastic deformation of the beam portion 22 even when it enters the gaps between the beam portion 22 and the extension portions 24.

As the resin layer 50, for example, the resin material may be molded on the upper surface 20m of the strain generating body 20 using a molding die, or a resin film may be laminated on the upper surface 20m of the strain generating body 20. The thickness of the resin layer 50 may be, for example, approximately from 10 um through 500 um. Since the resin layer 50 is formed along the upper surface 20m of the strain generating body 20, a load portion 53 covering the load portion 23 is formed on the resin layer 50. The load portion 53 projects from the upper surface of the resin layer 50. The amount of projection of the load portion 53 measured from the upper surface of the resin layer 50 is, for example, approximately 0.1 mm.

In the present disclosure, the strain gauge 100 is an example of a detector configured to detect a pulse wave. The strain gauge 100 is provided on the other surface of the strain generating body 20 positioned on the side opposite to the one surface. In the present embodiment, the other surface is a lower surface 20n. The strain gauge 100 can be provided, for example, on the lower surface side of the beam portion 22. Since the beam portion 22 is a flat plate shape, the strain gauge can be easily pasted on the beam portion 22. One or more strain gauges 100 may be provided, but in the present embodiment, four strain gauges 100 are provided. By providing four strain gauges 100, strain can be detected by a full bridge.

Two of the four strain gauges 100 are positioned on the beam having its longitudinal direction in the X-direction, at a portion of the beam close to the load portion 23 (at a portion on the center side of the circular opening), so that the two of the four strain gauges face each other across the load portion 23 in the plan view. The other two of the four strain gauges 100 are positioned on the beam having its longitudinal direction in the Y-direction, at a portion of the beam close to the base portion 21, so that the other two of the four strain gauges face each other across the load portion 23 in the plan view. With such an arrangement, compressive and tensile forces can be effectively detected and a large output can be obtained by the full bridge.

The pulse wave sensor 1 is used while being fixed to an arm of a subject so that the load portion 53 covering the load portion 23 contacts the radial artery of the subject. When a load is applied to the load portion 23 via the load portion 53 in response to a pulse wave of the subject and the beam portion 22 is elastically deformed, the resistance value of the resistor of the strain gauge 100 changes. The pulse wave sensor 1 can detect the pulse wave based on the change of the resistance value of the resistor of the strain gauge 100 caused by the deformation of the beam portion 22. The pulse wave is, for example, output as periodic voltage changes from a measuring circuit connected with an electrode of the strain gauge 100.

Since the pulse wave sensor 1 includes the resin layer 50 covering the upper surface 20m of the strain generating body 20 made of a metal, the strain generating body 20 made of the metal does not directly contact the skin of a subject. Thus, the subject can avoid developing cutaneous inflammation due to the metal or metal allergy.

If the gaps between the beam portion 22 and the extension portions 24 were opened to the outside of the pulse wave sensor 1, dust, foreign matter, or the like could get stuck in the gaps and the beam portion 22 could potentially become unable to elastically deform. However, for the pulse wave sensor 1, the resin layer 50 is also formed over the gaps between the beam portion 22 and the extension portions 24, and the gaps between the beam portion 22 and the extension portions 24 are not exposed to the outside of the pulse wave sensor 1. Thus, no dust, foreign matter, or the like will get stuck in the gaps between the beam portion 22 and the extension portions 24, and the pulse wave sensor 1 can perform more reliable and more stable pulse wave measurement.

In order for the pulse wave sensor 1 to detect a pulse wave of the subject, the pulse wave sensor 1 is required to have both sensitivity and rigidity. In the pulse wave sensor 1, the required sensitivity is that the output of the strain gauge 100 is 0.1 mV/V or more when a load of 1 g is applied to the load portion 23. When the sensitivity is less than 0.1 mV/V, a pulse wave cannot be clearly measured due to a decrease in the S/N ratio at the time of measurement. In addition, since a load of about 50 g is applied when the pulse wave sensor 1 is attached to the subject, the required rigidity is that the beam portion 22 does not cause plastic deformation when the load portion 23 is applied with the load of 50 g and a safety factor is set to 2. If this condition is not satisfied, it becomes difficult to use continuously as a pulse wave sensor.

The inventors found that the above sensitivity and rigidity can be compatible if the material of the strain generating body 20, the thickness t [mm], and the diameter d [mm] of the circular opening satisfy certain conditions. Specifically, the inventors experimentally determined the values of thickness t [mm] that can be compatible with the sensitivity and rigidity when the diameter d is 32 mm, 22 mm, 13 mm, and 7 mm using SUS (stainless steel), copper, and aluminum as the materials of the strain generating body 20.

The shape of the strain generating body 20 is as illustrated in FIGS. 2 and 3, and w₁ and w₂ are set to 3 mm. Note, however, that the resin layer 50 covering the strain generating body 20 is not provided in this experiment. In addition, four strain gauges 100 using a Cr mixed phase film described later as a resistor are attached to the positions illustrated in FIGS. 2 and 3.

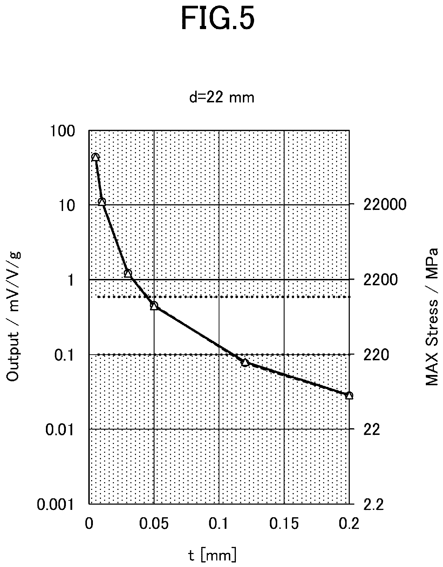

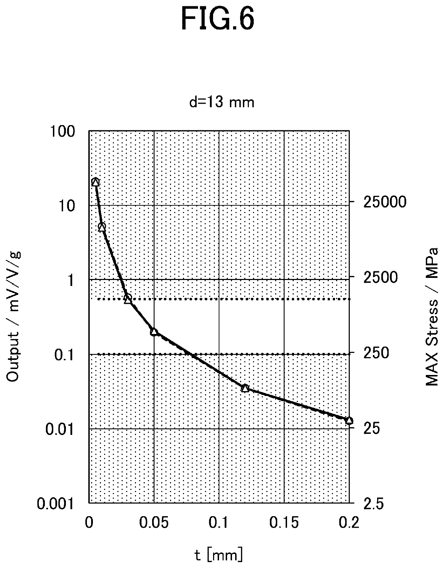

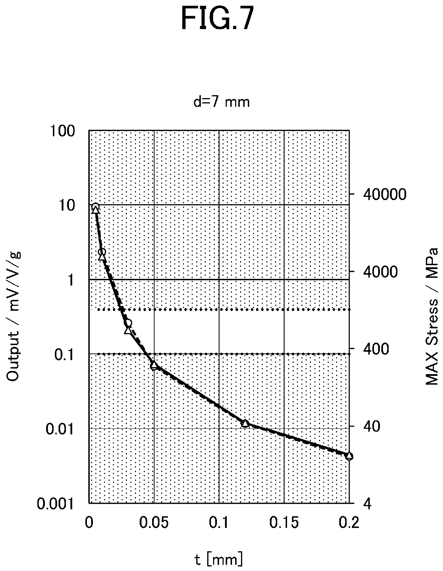

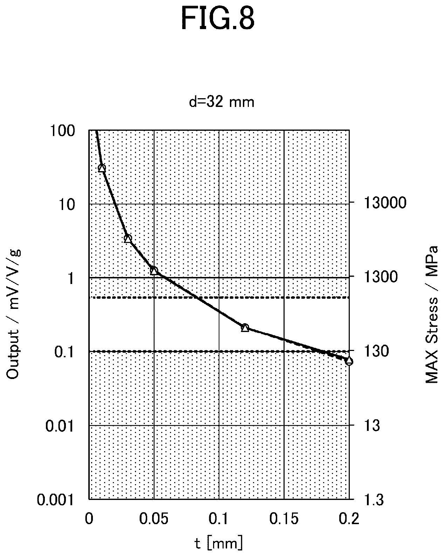

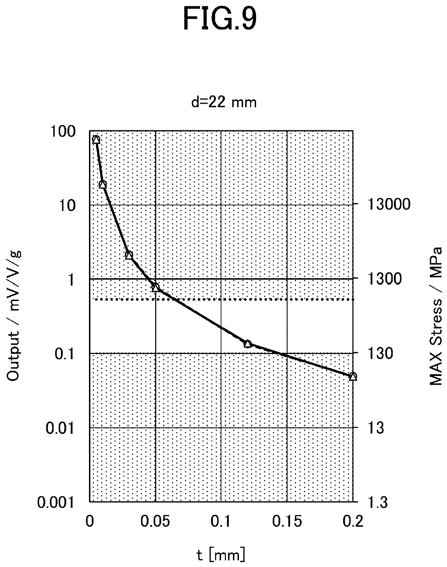

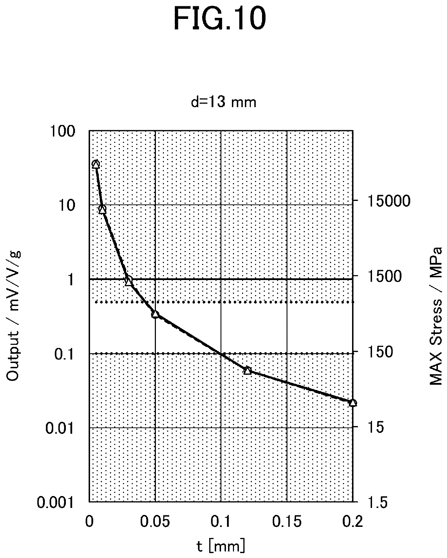

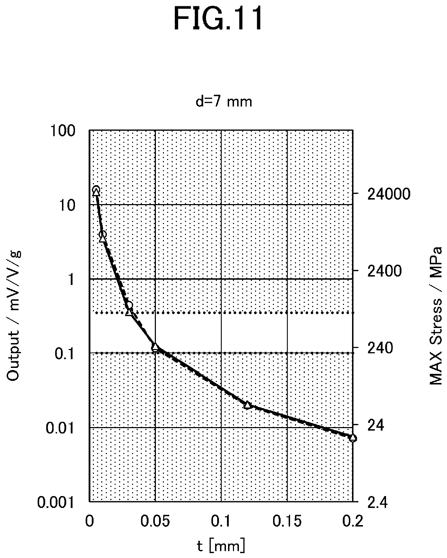

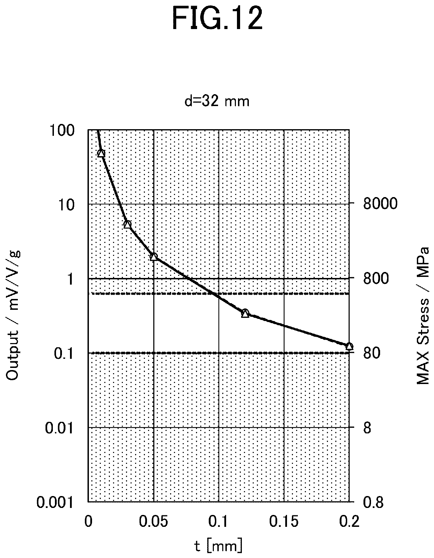

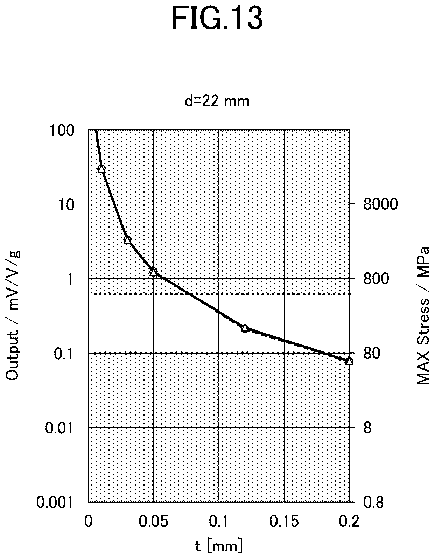

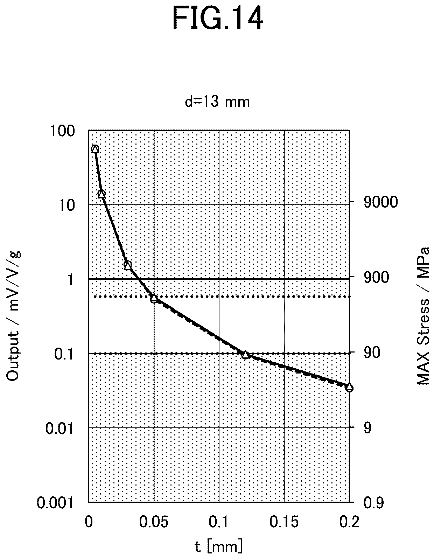

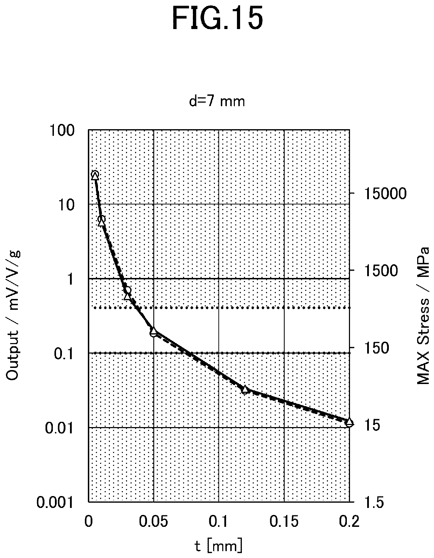

A graphical representation of the experimental results is illustrated in FIGS. 4 to 15. FIGS. 4 to 7 illustrate that SUS is used as the material of the strain generating body 20, FIGS. 8 to 11 illustrate that copper is used as the material of the strain generating body 20, and FIGS. 12 to 15 illustrate that aluminum is used as the material of the strain generating body 20. The left side of the vertical axis of each graph indicates the sensitivity, and the right side of the vertical axis indicates the maximum stress when a load of 50 g is applied to the load portion 23 and the safety factor is set to 2. The horizontal axis indicates the thickness t [mm] of the strain generating body 20.

In each graph, as the thickness t becomes thinner, the sensitivity becomes higher but the rigidity becomes lower, and the range of thickness t in which the sensitivity is 0.1 mV/V or more and the maximum stress is less than or equal to the yield strength (bearing capacity) is the range of thickness t in which the sensitivity and rigidity are compatible. In each graph, the range of thickness t in which the sensitivity and rigidity are compatible is the area indicated in white. The yield strength (bearing capacity) of SUS is 1275 MPa, that of copper is 675 MPa, and that of aluminum is 490 MPa.

**[Table 1]**

| Materials | d [mm] | tmin [mm] | Maximum Stress [Mpa] | tmax [mm] | Sensitivity [mV/V/g] |
|---|---|---|---|---|---|
| SUS | 32 | 0.059 | 1111 | 0.124 | 0.120 |
| | 22 | 0.046 | 1157 | 0.099 | 0.118 |
| | 13 | 0.030 | 1246 | 0.067 | 0.120 |
| | 7 | 0.026 | 909 | 0.034 | 0.148 |
| Copper | 32 | 0.084 | 553 | 0.166 | 0.116 |
| | 22 | 0.066 | 577 | 0.132 | 0.115 |
| | 13 | 0.044 | 662 | 0.088 | 0.121 |
| | 7 | 0.032 | 621 | 0.050 | 0.116 |
| Aluminum | 32 | 0.097 | 415 | 0.212 | 0.112 |
| | 22 | 0.079 | 405 | 0.168 | 0.112 |
| | 13 | 0.050 | 479 | 0.107 | 0.128 |
| | 7 | 0.038 | 449 | 0.063 | 0.116 |

Table 1 is a table that reads and summarizes the range of t that is compatible with sensitivity and rigidity. In Table 1, the diameter of the circular opening is d [mm] and the thickness of the strain generating body 20 is t [mm]. When the material of the strain generating body 20 is SUS and d is 32, the required range of t is 0.059≤t≤0.124 so as to have both sensitivity and rigidity. When the material of the strain generating body 20 is SUS and d is 22, the required range of t is 0.046≤t≤0.099. When the material of the strain generating body 20 is SUS and d is 13, the required range of t is 0.030≤t≤0.067. When the material of the strain generating body 20 is SUS and d is 7, the required range of t is 0.026≤t≤0.034.

When the material of the strain generating body 20 is copper and d is 32, the required range of t is 0.084≤t≤0.166. When the material of the strain generating body 20 is copper and d is 22, the required range of t is 0.066≤t≤0.132. When the material of the strain generating body 20 is copper and d is 13, the required range of t is 0.044≤t≤0.088. When the material of the strain generating body 20 is copper and d is 7, the required range of t is 0.032≤t≤0.050.

When the material of the strain generating body 20 is aluminum and d is 32, the required range of t is 0.097≤t≤0.212. When the material of the strain generating body 20 is aluminum and d is 22, the required range of t is 0.079≤t≤0.168. When the material of the strain generating body 20 is aluminum and d is 13, the required range of t is 0.050≤t≤0.107. When the material of the strain generating body 20 is aluminum and d is 7, the required range of t is 0.038≤t≤0.063.

Thus, if the material of the strain generating body 20, the thickness t [mm], and the diameter d [mm] of the circular opening satisfy certain conditions, the required sensitivity and rigidity can be compatible. In the case where the Cr mixed phase film is not used as the resistor of the strain gauge 100, the required sensitivity and rigidity cannot be compatible, or even if the sensitivity and rigidity are compatible, the allowable range of t becomes extremely narrow.

Hereinafter, the strain gauge 100 will be described.

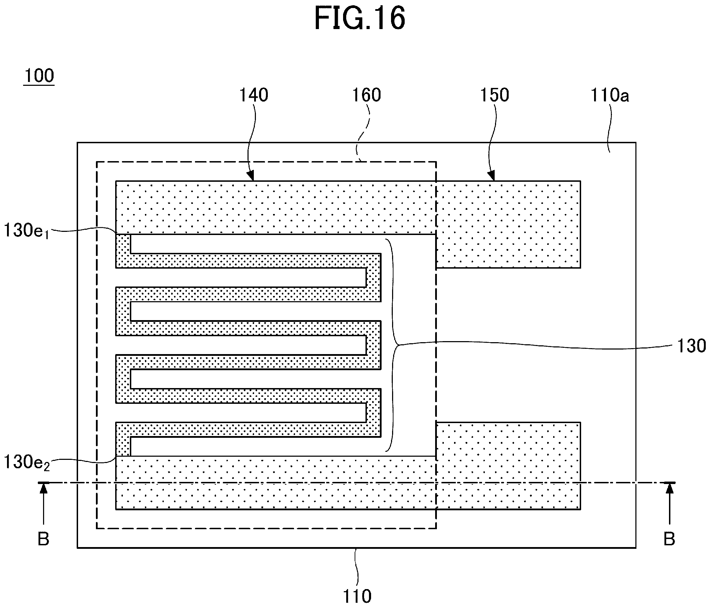

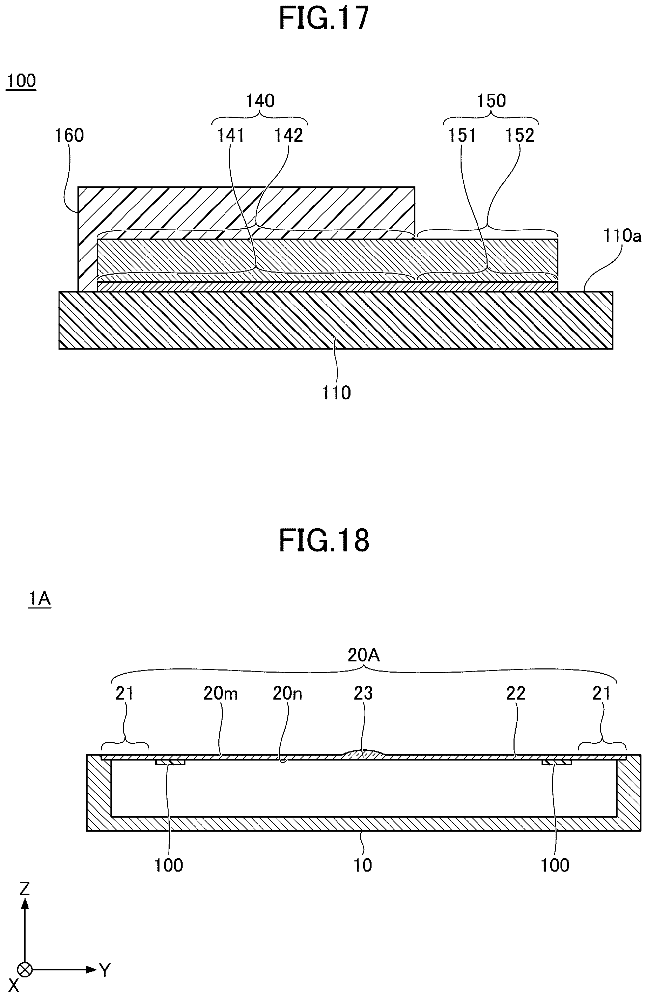

FIG. 16 is a plan view illustrating the strain gauge according to the first embodiment. FIG. 17 is a cross-sectional view illustrating a strain gauge according to the first embodiment, illustrating a cross-section along the line B-B of FIG. 16. Referring to FIGS. 16 and 17, the strain gauge 100 includes a substrate 110, a resistor 130, a wiring 140, an electrode 150, and a cover layer 160. In FIG. 16, only the outer edge of the cover layer 160 is illustrated as a dashed line for convenience. The cover layer 160 may be provided as necessary.

In FIGS. 16 and 17, for convenience, in the strain gauge 100, the side of the substrate 110 where the resistor 130 is provided is the upper side or one side, and the side where the resistor 130 is not provided is the lower side or the other side. In addition, the surface of each part on the side where the resistor 130 is provided is one surface or the upper surface, and the surface on the side where the resistor 130 is not provided is the other surface or the lower surface. However, the strain gauge 100 can be used in the reverse state or arranged at any angle. For example, in FIG. 2, the strain gauge 100 is attached to the beam portion 22 in the state of being upside down from FIG. 17. That is, the substrate 110 in FIG. 17 is attached to the lower surface of the beam portion 22 with an adhesive or the like. The plan view refers to an object viewed in the direction normal to the upper surface 110a of the substrate 110, and the planar shape refers to the shape of the object viewed in the direction normal to the upper surface 110a of the substrate 110.

The substrate 110 is a member that serves as a base layer for forming the resistor 130 and the like, and the substrate 110 has flexibility. The thickness of the substrate 110 is not particularly limited and can be appropriately selected according to the purpose, but can be, for example, in a range from 5 um to 500 um. In particular, when the thickness of the substrate 110 is in a range from 5 um to 200 um, it is preferable in terms of the transmissibility of strain from the surface of the strain generating body bonded to the lower surface of the substrate 110 via an adhesive layer or the like and also in terms of the dimensional stability with respect to the environment. When the thickness of the substrate 110 is 10 um or more, it is more preferable in terms of the insulating property.

The substrate 110 can be formed from, for example, an insulating resin film such as a polyimide (PI) resin, an epoxy resin, a polyetheretherketone (PEEK) resin, a polyethylene naphthalate (PEN) resin, a polyethylene terephthalate (PET) resin, a polyphenylene sulfide (PPS) resin, a liquid crystal polymer (LCP) resin, a polyolefin resin, and the like. The film refers to a member having a thickness of about 500 um or less and having flexibility.

The term "formed from an insulating resin film" here does not prevent the substrate 110 from containing fillers, impurities or the like in the insulating resin film. The substrate 110 may be formed from, for example, an insulating resin film containing a filler such as silica, alumina, or the like.

Materials other than the resin for the substrate 110 include, for example, crystalline materials such as SiO₂, ZrO₂ (including YSZ), Si, Si₂N₃, Al₂O₃ (including sapphire), ZnO, perovskite-based ceramics (CaTiO₃, BaTiO₃), or the like, and further include amorphous glass or the like. Metals such as aluminum, aluminum alloy (duralumin), titanium, or the like may be used as materials for the substrate 110. In this case, for example, an insulating film is formed on the substrate 110 made of metal.

The resistor 130 is a thin film formed in a predetermined pattern on the substrate 110. The resistor 130 is a sensitive part that causes a resistance change when being strained. The resistor 130 may be formed directly on the upper surface 110a of the substrate 110, or through other layers on the upper surface 110a of the substrate 110. In FIG. 16, the resistor 130 is illustrated in a dark-colored pear-skin pattern for convenience.

The resistor 130 has a structure in which a plurality of thin and long portions are arranged at predetermined intervals with the longitudinal direction set in the same direction (the direction of the line B-B in FIG. 16), and the end portions of adjacent thin and long portions are connected on the alternate sides, and folded back in a zigzag manner as a whole. The longitudinal direction of the plurality of thin and long portions is a grid direction, and the direction perpendicular to the grid direction is a grid width direction (the direction perpendicular to the line B-B in FIG. 16).

The end portions, on the longitudinal-direction one side, of the two thin and long portions that are located on the outermost side in the grid width direction are bent in the grid width direction to form respective ends 130e₁ and 130e₂ in the grid width direction of the resistor 130. The respective ends 130e₁ and 130e₂ in the grid width direction of the resistor 130 are electrically connected to the electrodes 150 via the wiring 140. In other words, the wiring 140 electrically connects the respective ends 130e₁ and 130e₂ in the grid width direction of the resistor 130 with the respective electrodes 150.

The resistor 130 can be formed, for example, from a material containing chromium (Cr), a material containing nickel (Ni), or a material containing both Cr and Ni. That is, the resistor 130 can be formed from a material containing at least one of Cr and Ni. The material containing Cr includes, for example, a Cr mixed phase film. The material containing Ni includes, for example, copper nickel (Cu-Ni). The material containing both Cr and Ni includes, for example, nickel chromium (Ni-Cr).

Here, the Cr mixed phase film is a film in which Cr, CrN, Cr₂N, and the like are mixed. The Cr mixed phase film may contain unavoidable impurities such as chromium oxide and the like.

The thickness of the resistor 130 is not particularly limited and can be appropriately selected according to the purpose, but can be, for example, in a range from about 0.05 um to 2 um. In particular, when the thickness of the resistor 130 is 0.1 um or more, it is preferable in that the crystallinity (for example, the crystallinity of α-Cr) of the crystals constituting the resistor 130 is improved. When the thickness of the resistor 130 is 1 um or less, it is more preferable in that film cracks caused by the internal stress of the film constituting the resistor 130 and warpage from the substrate 110 can be reduced. The width of the resistor 130 can be set to about in a range from 10 um to 100 um if the required specifications such as the resistance value and the lateral sensitivity are optimized and the wire breakage countermeasures are considered.

For example, when the resistor 130 is a Cr mixed phase film, the stability of the gauge characteristic can be improved by using alpha chromium (α-Cr), which is a stable crystalline phase, as the main component. Moreover, when the resistor 130 uses α-Cr as the main component, the gauge rate of the strain gauge 100 can be 10 or more, and the temperature coefficient of strain gauge rate TCS and the temperature coefficient of resistance TCR can be set within a range from - 1000 ppm/°C to +1000 ppm/°C. Here, the main component means that the target material occupies 50% by weight or more of the total material constituting the resistor, but from the viewpoint of improving the gauge characteristics, the resistor 130 preferably contains 80% by weight or more of α-Cr, and more preferably 90% by weight or more. α-Cr is Cr having a bcc structure (body-centered cubic lattice structure).

When the resistor 130 is a Cr mixed phase film, CrN and Cr₂N contained in the Cr mixed phase film are preferably 20% by weight or less. When CrN and Cr₂N contained in the Cr mixed phase film are 20% by weight or less, the lowering of the gauge ratio can be suppressed.

The proportion of Cr₂N in CrN and Cr₂N is preferably 80% by weight or more and less than 90% by weight, and more preferably 90% by weight or more and less than 95% by weight. When the proportion of Cr₂N in CrN and Cr₂N is 90% by weight or more and less than 95% by weight, the decrease in TCR (negative TCR) becomes more remarkable due to Cr₂N having semiconducting properties. In addition, by reducing ceramization, brittle fracture is reduced.

On the other hand, when a small amount of N₂ or atomic N is mixed or present in the film, N₂ or atomic N escapes from the film to the outside of the film due to an external environment (for example, a high-temperature environment), resulting in a change in film stress. By creating chemically stable CrN, a stable strain gauge without generating the unstable N can be obtained.

The wiring 140 is formed on the substrate 110 and is electrically connected to the resistor 130 and the electrodes 150. The wiring 140 has a first metal layer 141 and a second metal layer 142 laminated on the upper surface of the first metal layer 141. The wiring 140 is not limited to a straight line and can be formed in any pattern. The wiring 140 can be any width and any length. In FIG. 16, for convenience, the wiring 140 and the electrodes 150 are illustrated in a lighter-colored pear-skin pattern than the resistor 130.

The electrode 150 is formed on the substrate 110 and is electrically connected to the resistor 130 via the wiring 140, and is formed in a substantially rectangular shape, for example, wider than the wiring 140. The electrode 150 is a pair of electrodes for outputting a change in the resistance value of the resistor 130 caused by strain to the outside, and a lead wire or the like for external connection is joined, for example.

The electrode 150 has a pair of first metal layers 151 and a second metal layer 152 laminated on the upper surface of each first metal layer 151. The first metal layers 151 are electrically connected to the ends 130e₁ and 130e₂ of the resistor 130 through the first metal layers 141 of the wiring 140. The first metal layer 151 is formed in a substantially rectangular shape in a plan view. The first metal layer 151 may be formed in the same width as the wiring 140.

Although the resistor 130, the first metal layer 141, and the first metal layer 151 are designated separately for convenience, they can be integrally formed of the same material in the same process. Accordingly, the resistor 130, the first metal layer 141, and the first metal layer 151 have substantially the same thickness. In addition, although the second metal layer 142 and the second metal layer 152 are designated separately for convenience, they can be integrally formed of the same material in the same process. Therefore, the thickness of the second metal layer 142 and the second metal layer 152 is substantially the same.

The second metal layers 142 and 152 are formed from a material having a lower resistance than the resistor 130 (the first metal layers 141 and 151). The material of the second metal layers 142 and 152 is not particularly limited if the material has a lower resistance than the resistor 130, and can be appropriately selected according to the purpose. For example, if the resistor 130 is a Cr mixed phase film, the material of the second metal layers 142 and 152 may include Cu, Ni, Al, Ag, Au, Pt, or an alloy of any of these metals, a compound of any of these metals, or a laminated film suitably laminated with any of these metals, alloys, or compounds. The thickness of the second metal layers 142 and 152 is not particularly limited and may be suitably selected according to the purpose, but may be, for example, in a range from 3 um to 5 um.

The second metal layers 142 and 152 may be formed on a part of the upper surfaces of the first metal layers 141 and 151, or may be formed on the entire upper surfaces of the first metal layers 141 and 151. One or more other metal layers may be laminated on the upper surface of the second metal layer 152. For example, the second metal layer 152 may be a copper layer, and a gold layer may be laminated on the upper surface of the copper layer. Alternatively, the second metal layer 152 may be a copper layer, and a palladium layer and a gold layer may be successively laminated on the upper surface of the copper layer. By making the top layer of the electrode 150 a gold layer, the solder wettability of the electrode 150 can be improved.

As described above, the wiring 140 has a structure in which the second metal layer 142 is laminated on the first metal layer 141 made of the same material as the resistor 130. Therefore, since the wiring 140 has a lower resistance than the resistor 130, it is possible to suppress the wiring 140 from functioning as a resistor. As a result, the strain detection accuracy by the resistor 130 can be improved.

In other words, by providing the wiring 140 with a lower resistance than the resistor 130, the substantially sensitive portion of the strain gauge 100 can be limited to a local region where the resistor 130 is formed. Therefore, the strain detection accuracy by the resistor 130 can be improved.

In particular, in a highly sensitive strain gauge with a gauge ratio of 10 or more using a Cr mixed phase film as the resistor 130, limiting the substantially sensitive portion to the local region where the resistor 130 is formed by making the wiring 140 less resistant than the resistor 130 has a remarkable effect on improving the strain detection accuracy. In addition, making the wiring 140 less resistant than the resistor 130 also has an effect on reducing the lateral sensitivity.

The cover layer 160 is formed on the substrate 110, covers the resistor 130 and the wiring 140, and exposes the electrode 150. A portion of the wiring 140 may be exposed from the cover layer 160. By providing the cover layer 160 covering the resistor 130 and the wiring 140, mechanical damage or the like can be prevented from occurring to the resistor 130 and the wiring 140. Further, by providing the cover layer 160, the resistor 130 and the wiring 140 can be protected from moisture or the like. The cover layer 160 may be provided so as to cover the entire portion excluding the electrode 150.

The cover layer 160 may be formed of, for example, an insulating resin such as a PI resin, an epoxy resin, a PEEK resin, a PEN resin, a PET resin, a PPS resin, or a composite resin (for example, silicone resin, polyolefin resin). The cover layer 160 may contain fillers or pigments. The thickness of the cover layer 160 is not particularly limited and can be appropriately selected according to the purpose, but can be, for example, about in a range from 2 um to 30 um.

In order to manufacture the strain gauge 100, first, a substrate 110 is prepared and a metal layer (designated as a metal layer A for convenience) is formed on the upper surface 110a of the substrate 110. The metal layer A is a layer which is finally patterned into the resistor 130, the first metal layer 141, and the first metal layer 151. Accordingly, the material and thickness of the metal layer A are the same as those of the resistor 130, the first metal layer 141, and the first metal layer 151.

The metal layer A can be formed by, for example, a magnetron sputtering method by using a raw material capable of forming the metal layer A as a target. Instead of the magnetron sputtering method, the metal layer A may be formed by a reactive sputtering method, a vapor deposition method, an arc ion plating method, a pulsed laser deposition method, or the like.

From the viewpoint of stabilizing the gauge characteristics, a functional layer with a predetermined thickness may preferably be formed on the upper surface 110a of the substrate 110 as a foundation layer by, for example, a conventional sputtering method before forming the metal layer A.

In the present invention, the functional layer refers to a layer having a function of promoting crystal growth of at least the metal layer A (resistor 130) which is at least an upper layer thereof. In addition, the functional layer preferably has a function of preventing oxidation of the metal layer A by oxygen and moisture contained in the substrate 110 and a function of improving adhesion between the substrate 110 and the metal layer A. The functional layer may further have other functions.

Since the insulating resin film constituting the substrate 110 contains oxygen and moisture, especially when the metal layer A contains Cr, Cr forms an auto-oxide film. Therefore, providing the functional layer to prevent oxidation of the metal layer A is effective.

The material of the functional layer is not particularly limited and can be suitably selected according to the purpose as long as the material has a function of promoting crystal growth of at least the metal layer A (resistor 130) which is an upper layer. Examples of the material of the functional layer include metals selected from the group consisting of chromium (Cr), titanium (Ti), vanadium (V), niobium (Nb), tantalum (Ta), nickel (Ni), yttrium (Y), zirconium (Zr), hafnium (Hf), silicon (Si), carbon (C), zinc (Zn), copper (Cu), bismuth (Bi), iron (Fe), molybdenum (Mo), tungsten (W), ruthenium (Ru), rhodium (Rh), rhenium (Re), osmium (Os), iridium (Ir), platinum (Pt), palladium (Pd), silver (Ag), gold (Au), cobalt (Co), manganese (Mn), and aluminum (Al). One type of the metal selected from the group or two or more types of the metal selected from the group can be used. An alloy of any metals selected from the group or a compound of any metals selected from the group may also be used.

Examples of the alloys include FeCr, TiAl, FeNi, NiCr, CrCu, and the like. Examples of the compounds include TiN, TaN, Si₃N₄, TiO₂, Ta₂O₅, SiO₂, and the like.

When the functional layer is formed from a conductive material such as a metal or alloy, the thickness of the functional layer is preferably 1/20 or less of the thickness of the resistor. In such a range, crystal growth of α-Cr can be promoted, and it is possible to prevent the strain detection sensitivity from being reduced due to a part of the current, which is flowing through the resistor, flowing to the functional layer.

When the functional layer is formed from a conductive material such as a metal or alloy, the thickness of the functional layer is preferably 1/50 or less of the thickness of the resistor. In such a range, the crystal growth of α-Cr can be promoted, and it is possible to further prevent the detection sensitivity of the strain from being lowered due to a part of the current, which is flowing through the resistor, flowing to the functional layer.

When the functional layer is formed from a conductive material such as a metal or alloy, the thickness of the functional layer is more preferably 1/100 or less of the thickness of the resistor. In such a range, it is possible to yet further prevent the detection sensitivity of the strain from being lowered due to a part of the current, which is flowing through the resistor, flowing to the functional layer.

When the functional layer is formed from an insulating material such as an oxide or nitride, the thickness of the functional layer is preferably in a range from 1 nm to 1 um. In this range, crystal growth of α-Cr can be promoted, and the film can be easily formed without cracks in the functional layer.

When the functional layer is formed from an insulating material such as an oxide or nitride, the thickness of the functional layer is more preferably in a range from 1 nm to 0.8 um. In this range, crystal growth of α-Cr can be promoted and the film can be formed more easily without cracks in the functional layer.

If the functional layer is formed from an insulating material such as an oxide or nitride, the thickness of the functional layer is more preferably in a range from 1 nm to 0.5 um. In this range, crystal growth of α-Cr can be promoted and the film can be formed more easily without cracks in the functional layer.

The planar shape of the functional layer is patterned almost identically to the planar shape of the resistor illustrated in FIG. 16, for example. However, the planar shape of the functional layer is not limited to the case where it is almost identical to the planar shape of the resistor. In the case where the functional layer is formed from an insulating material, it is not necessary to pattern it in the same shape as the planar shape of the resistor. In this case, the functional layer may be formed in a solid shape at least in the region where the resistor is formed. Alternatively, the functional layer may be formed in a solid pattern over the entire upper surface of the substrate 110.

Further, when the functional layer is formed from an insulating material, the thickness of the functional layer is formed relatively thick, in a range from 50 nm to 1 um, and in a solid pattern, which increases the thickness and surface area of the functional layer, allowing the heat generated by the resistor to dissipate to the substrate 110. As a result, in the strain gauge 100, a decrease in measurement accuracy due to self-heating of the resistor can be suppressed.

For example, the functional layer can be formed in vacuum by a conventional sputtering method in which argon (Ar) gas is introduced into a chamber by using a raw material capable of forming the functional layer as a target. By using the conventional sputtering method, the functional layer is formed while etching the upper surface 110a of the substrate 110 with Ar, so that the adhesion improvement effect can be obtained by minimizing the film formation amount of the functional layer.

However, this is an example of the film formation method of the functional layer, and the functional layer may be formed by other methods. For example, the adhesion improvement effect may be obtained by activating the upper surface 110a of the substrate 110 by plasma treatment using Ar or the like before the film formation of the functional layer, and then the functional layer may be formed in vacuum by a magnetron sputtering method.

The combination of the material of the functional layer and the material of the metal layer A is not particularly limited and can be appropriately selected according to the purpose, but, for example, a Cr mixed phase film in which α-chromium (α-Cr) is the main component can be formed as the metal layer A, using Ti as the functional layer.

In this case, for example, the metal layer A can be formed by a magnetron sputtering method in which Ar gas is introduced into the chamber by using a raw material capable of forming a Cr mixed phase film as a target. Alternatively, the metal layer A can be formed by a reactive sputtering method in which an appropriate amount of nitrogen gas is introduced into the chamber together with Ar gas by using pure Cr as a target. At this time, the proportion of CrN and Cr₂N contained in the Cr mixed phase film and the proportion of Cr₂N in CrN and Cr₂N can be adjusted by changing the amount of nitrogen gas introduced and the pressure (nitrogen partial pressure) or by adjusting the heating temperature by providing a heating step.

In these methods, the growth surface of the Cr mixed phase film is determined by the functional layer formed from Ti, and the Cr mixed phase film mainly composed of α-Cr, which is a stable crystal structure, can be formed. In addition, the Ti constituting the functional layer diffuses into the Cr mixed phase film, thereby improving the gauge characteristics. For example, the gauge rate of the strain gauge 100 may be 10 or more, and the temperature coefficient of strain gauge TCS and the temperature coefficient of resistance TCR may be within a range from -1000 ppm/°C to +1000 ppm/°C. When the functional layer is formed from Ti, Ti or titanium nitride (TiN) may be included in the Cr mixed phase film.

In the case where the metal layer A is the Cr mixed phase film, the functional layer formed from Ti has a function of promoting crystal growth of the metal layer A, a function of preventing oxidation of the metal layer A by oxygen and moisture contained in the substrate 110, and a function of improving adhesion between the substrate 110 and the metal layer A. The same applies when Ta, Si, Al, and Fe are used instead of Ti as the functional layer.

Thus, by providing the functional layer in the lower layer of the metal layer A, the crystal growth of the metal layer A can be promoted, and the metal layer A composed of the stable crystal phase can be produced. As a result, the stability of the gauge characteristics can be improved in the strain gauge 100. Further, by diffusing the material constituting the functional layer into the metal layer A, the gauge characteristics can be improved in the strain gauge 100.

Next, the second metal layer 142 and the second metal layer 152 are formed on the upper surface of the metal layer A. The second metal layer 142 and the second metal layer 152 can be formed by, for example, a photolithography method.

Specifically, a seed layer is first formed to cover the upper surface of the metal layer A by, for example, a sputtering method or an electroless plating method. Next, a photosensitive resist is formed on the entire surface of the upper surface of the seed layer, and an opening is formed to expose an area for forming the second metal layer 142 and the second metal layer 152 by exposure and development. At this time, the pattern of the second metal layer 142 can be formed so as to be a desirable shape by adjusting the shape of the opening of the resist. For example, a dry film resist or the like can be used as the resist.

Next, for example, a second metal layer 142 and a second metal layer 152 are formed on the seed layer exposed in the opening by an electrolytic plating method using the seed layer as a power supply path. The electrolytic plating method is suitable in that the tact is high and a low-stress electrolytic plating layer can be formed as the second metal layer 142 and the second metal layer 152. By making the thick electrolytic plating layer low-stress, warping of the strain gauge 100 can be prevented. The second metal layer 142 and the second metal layer 152 may be formed by a non-electrolytic plating method.

Next, the resist is removed. The resist can be removed, for example, by dipping the resist material into a dissolvable solution.

A photosensitive resist is then formed on the entire surface of the upper surface of the seed layer, exposed and developed, and patterned into a planar shape similar to that of the resistor 130, wiring 140, and electrode 150 of FIG. 16. For example, a dry film resist and the like can be used as the resist. Then, using the resist as an etching mask, the metal layer A and the seed layer exposed from the resist are removed to form the planar resistor 130, the wiring 140, and the electrode 150 illustrated in FIG. 16.

For example, unnecessary portions of the metal layer A and the seed layer can be removed by wet etching. If a functional layer is formed under the metal layer A, the functional layer is patterned by etching into the planar shape illustrated in FIG. 16 in the same manner as the resistor 130, the wiring 140, and the electrode 150. At this point, a seed layer is formed on the resistor 130, the first metal layer 141, and the first metal layer 151.

Next, the second metal layer 142 and the second metal layer 152 are formed by using the second metal layer 142 and the second metal layer 152 as etching masks and removing unnecessary seed layers exposed from the second metal layer 142 and the second metal layer 152. The seed layers immediately below the second metal layer 142 and the second metal layer 152 remain. For example, the unnecessary seed layer can be removed by wet etching using an etchant in which the seed layer is etched and the functional layer, the resistor 130, the wiring 140, and the electrode 150 are not etched.

Then, if necessary, a cover layer 160 covering the resistor 130 and the wiring 140 and exposing the electrode 150 is provided on the upper surface 110a of the substrate 110 to form the strain gauge 100. For example, the cover layer 160 can be formed by laminating a semi-cured thermosetting insulating resin film on the upper surface 110a of the substrate 110 so as to cover the resistor 130 and the wiring 140 and expose the electrode 150, and curing the film by heating. The cover layer 160 can be prepared by coating the upper surface 110a of the substrate 110 with a liquid or paste thermosetting insulating resin covering the resistor 130 and the wiring 140 and exposing the electrode 150, and heating to cure the resin.

### <Modified examples of the first embodiment>

In a modified example of the first embodiment, an example of a pulse wave sensor using a nonmetallic strain generating body will be set forth. In the modified example of the first embodiment, description of the same components as those in the embodiment described above may be omitted.

FIG. 18 is a cross-sectional view (No. 1) illustrating the pulse wave sensor according to the modified example of the first embodiment. A pulse wave sensor 1A illustrated in FIG. 18 includes a strain generating body 20A made of a nonmetallic material. As the material of the strain generating body 20A, for example, ceramics, glass, and the like may be used. The strain generating body 20A is the same as the strain generating body 20 except that a nonmetallic material is used. The pulse wave sensor 1A includes no resin layer 50, unlike the pulse wave sensor 1.

As in the pulse wave sensor 1A, the strain generating body 20A may be made of a nonmetallic material. In this case, it is unnecessary to provide a resin layer 50, because a subject will not develop cutaneous inflammation due to a metal or metal allergy even if the strain generating body 20A directly contacts the subject's skin.

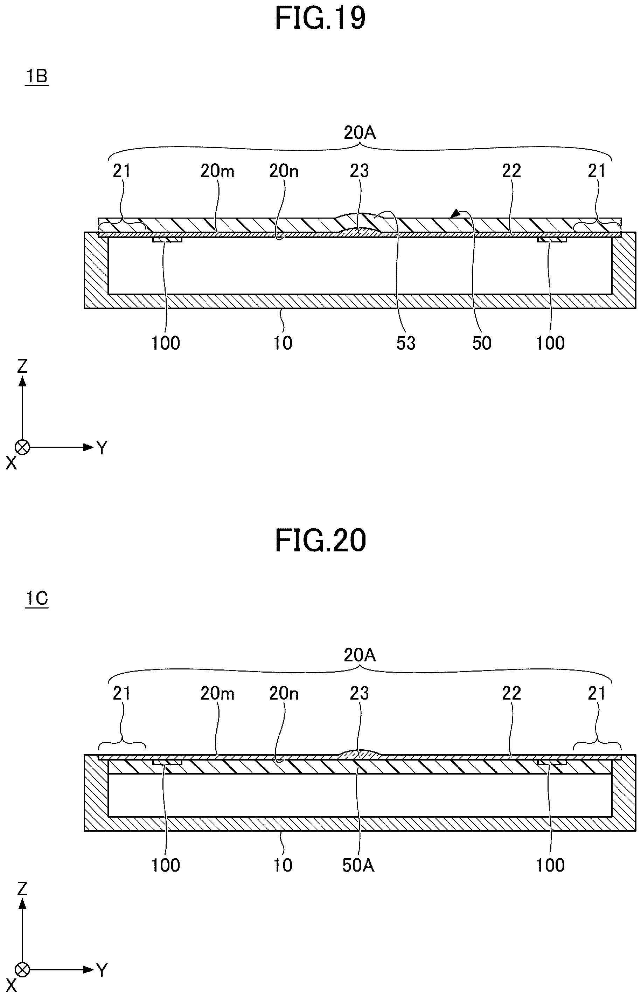

FIG. 19 is a cross-sectional view (No. 2) illustrating a pulse wave sensor according to a modified example of the first embodiment. Also when a nonmetallic strain generating body 20A is used as in a pulse wave sensor 1B illustrated in FIG. 19, a resin layer 50 covering an upper surface 20m of the strain generating body 20A may be provided as in the pulse wave sensor 1 illustrated in FIG. 3. The resin layer 50 is also formed over gaps between a beam portion 22 and extension portions 24, and the gaps between the beam portion 22 and the extension portions 24 are not exposed to the outside of the pulse wave sensor 1B. Thus, no dust, foreign matter, or the like will get stuck in the gaps between the beam portion 22 and the extension portions 24, and the pulse wave sensor 1B can perform more reliable and more stable pulse wave measurement.

FIG. 20 is a cross-sectional view (No. 3) illustrating a pulse wave sensor according to a modified example of the first embodiment. When a nonmetallic strain generating body 20A is used as in a pulse wave sensor 1C illustrated in FIG. 20, a resin layer 50A covering a lower surface 20n of the strain generating body 20A may be provided instead of providing a resin layer 50 covering an upper surface 20m of the strain generating body 20A. The material and thickness of the resin layer 50A are the same as those of the resin layer 50. In the pulse wave sensor 1C, the resin layer 50A enters gaps between a beam portion 22 and extension portions 24, and fills the gaps between the beam portion 22 and the extension portions 24. Thus, no dust, foreign matter, or the like will get stuck in the gaps between the beam portion 22 and the extension portions 24, and the pulse wave sensor 1C can perform more reliable and more stable pulse wave measurement. In addition, together with a cover layer 160, the resin layer 50A protects a strain gauge 100 from moisture and the like.

It is optional to provide the resin layer 50 covering the upper surface 20m of the strain generating body 20A, and to also provide the resin layer 50A covering the lower surface 20n of the strain generating body 20A. Moreover, a resin layer 50A may further be provided on the lower surface 20n of the strain generating body 20 illustrated in FIGS. 1 to 3. In this case, together with the cover layer 160, the resin layer 50A protects the strain gauge 100 from moisture and the like.

### <Second embodiment>

In the embodiment described above and the modified examples thereof, an example in which the detector according to the present disclosure is a strain gauge using a resistor has been described. That is, in the embodiment described above, a case in which the detector according to the present disclosure is an electric resistance-based metallic strain gauge has been described. However, the detector according to the present disclosure is not limited to a metallic strain gauge. For example, the detector according to the present disclosure may be a strain gauge configured to detect a magnetic change caused by a strain of a strain generating body (or a structure equivalent to the strain generating body), by using a detecting element included in the strain gauge.

Specifically, the detector according to the present disclosure may be a strain gauge including a detecting element utilizing the Villari effect (described below). The detector according to the present disclosure may be a strain gauge including a detecting element having a magnetic tunnel junction (described below) structure. In the following second embodiment, a strain gauge including a detecting element utilizing the Villari effect will be described. In the third embodiment, a strain gauge including a detecting element having a magnetic tunnel junction structure will be described.

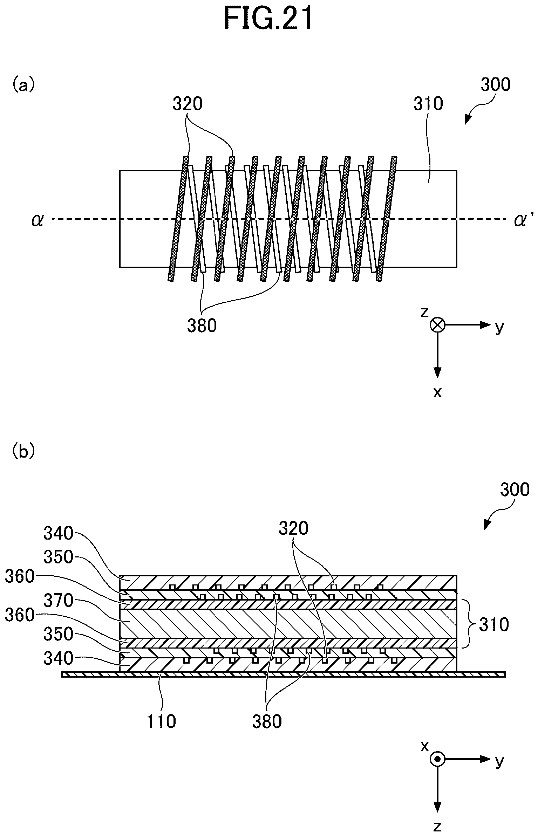

In the embodiments of the present specification, the components having the same functions will be denoted by the same names and component numbers, and description of such components will not be repeated. The directions of the x-axis, the y-axis, and the z-axis in the drawings relating to the following embodiments (the drawings from FIG. 21) are the same as the directions of the x-axis, the y-axis, and the z-axis indicated in FIG. 2 and FIG. 3. In addition, in the following description, the z-axis positive direction will be referred to as "upper", and the z-axis negative direction will be referred to as "lower". That is, in the following description, the "upper side" is the z-axis positive direction side, and an "upper surface" is a surface positioned on the z-axis positive direction side. The "lower side" is the z-axis negative direction side, and a "lower surface" is a surface positioned on the z-axis negative direction side.

FIG. 21 is a diagram illustrating an example of a detecting element 300 included in a strain gauge according to the second embodiment. FIG. 21 (a) is a plan view of the detecting element 300 viewed from the z-axis negative direction side in the positive direction (i.e., from a lower surface to an upper surface). FIG. 21 (b) is a cross-sectional view of the detecting element 300 illustrated in FIG. 21 (a), taken along a straight line α-α'. FIG. 21 (a) and (b) illustrate no wirings extending from the detecting element 300. However, a wiring for connecting a drive coil 320 described below and a power source with each other, and a wiring for transmitting a current detected by a sensing coil 380 may be connected to the detecting element 300.

As illustrated in FIG. 21 (a), the detecting element 300 includes a drive coil 320, a sensing coil 380, and a base layer 310. The base layer 310 is a layer serving as a core material of the drive coil 320 and the sensing coil 380. The sensing coil 380 is a coil configured to detect the magnetization magnitude of the base layer 310 (more strictly, a base metal 370 described below). The drive coil 320 is a coil configured to generate a magnetic field. The detecting element 300 has a double structure in which the sensing coil 380 is wound on the inner side and the drive coil 320 is wound on the outer side around the base layer 310 serving as a core material. The material of the drive coil 320 and the sensing coil 380 is preferably a conductive metal such as Cu, Ag, Al, Au, and the like, and alloys of these metals. The number of turns and the cross-sectional area of the drive coil 320 and the sensing coil 380 may be appropriately designed in accordance with the strain sensitivity required of the detecting element 300.

As will be described in detail below, when a stress is applied to the base layer 310, the magnetization magnitude of the base metal 370 (described below) included in the base layer 310 changes. The detecting element 300 can determine the magnitude of the stress applied to the base layer 310 (i.e., the straining degree), by detecting this change in the magnetization magnitude by using the sensing coil 380.

With reference to the cross-sectional view of FIG. 21 (b), the configuration of the detecting element 300 will further be described. In FIG. 21 (b), the drive coil 320, the sensing coil 380, and three insulating layers 340, 350, and 360 are formed to surround the base metal 370 serving as a core material. That is, any layers denoted by the same component number in FIG. 21 (b) are continuous around the base metal 370.

The base metal 370 is a member serving as the core material of the various coils and the insulating layers. The base metal 370 may be, for example, an approximately flat plate-shaped metal plate. The base metal 370 is covered with the insulating layer 360 in a surrounding manner. It is preferable to form the base metal 370 using, for example, a soft magnetic material such as a Fe-Si-Al-based alloy such as sendust and the like, and a Ni-Fe-based alloy such as permalloy and the like. The base layer 310 described above is made of the base metal 370 and the insulating layer 360 as illustrated in FIG. 21 (b).

The insulating layer 350 is formed on the outer side of the insulating layer 360 so as to surround the insulating layer 360. An insulating layer 340 is further formed on the outer side of the insulating layer 350. The insulating layer 350 is a layer including the sensing coil 380, and is a layer in which the gaps in the sensing coil 380 are filled with an insulating material. The insulating layer 340 is a layer including the drive coil 320, and is a layer in which the gaps in the drive coil 320 are filled with an insulating material. It is preferable that the insulating layers 340, 350, and 360 are cured products of resists such as dry film, photosensitive polyimide, and the like that do not affect a magnetic field.

One surface of the detecting element 300 may be pasted on a substrate 110 as illustrated in FIG. 21 (b). The substrate 110 is a member on which the detecting element 300 is fixed. For example, the substrate 110 may be a flexible substrate made of a plastic film or the like. The detecting element 300 is pasted on the strain generating body 20 or 20A via the substrate 110. The detecting element 300 may be a detecting element having a flat plate shape or a thin film shape as a whole. The detecting element 300 having a flat plate shape or a thin film shape is easier to paste on the substrate 110. The substrate 110 is not an indispensable component of the detecting element 300. For example, the detecting element 300 may be used with its upper surface directly pasted on the strain generating body 20 or 20A, instead of the substrate 110 being provided to the detecting element 300.

The strain generating bodies 20 and 20A according to the present embodiment may be basically of the same configuration and material as those of the strain generating bodies 20 and 20A according to the first embodiment. However, it is more preferable that the strain generating bodies 20 and 20A are made of a nonmagnetic body. The strain generating bodies 20 and 20A according to the present embodiment may be made of, for example, a nonmagnetic stainless steel.

Next, the principle by which a strain is detected by using the detecting element 300 will be roughly described. The detecting element 300 includes the base metal 370, which is a magnetic body. When an alternating current is supplied from a power source to the drive coil 320, the drive coil 320 generates an altermatic magnetic field around itself. As a result, a magnetic field is generated, and the base metal 370 is magnetized. When the strain generating body 20 or 20A deforms in this state, a strain is generated. The strain is transmitted via the substrate 110 to apply a stress to the base metal 370. When the detecting element 300 is pasted on the strain generating body 20 or 20A via no substrate 110, the stress is transmitted from the strain generating body 20 or 20A directly to the base metal 370 (and the insulating layers 340 to 360 covering it).

When a stress is applied to the base metal 370, the magnetic permeability of the base metal 370 changes in accordance with the stress. Thus, the magnetization magnitude (magnetization degree) of the base metal 370 changes. The phenomenon, in which application of a stress to a magnetic body causes changes in the magnetic permeability and the magnetization magnitude of the magnetic body, is referred to as the "Villari effect". According to the configuration of the detecting element 300, an alternating voltage corresponding to the magnetization magnitude of the base metal 370 is induced across the sensing coil 380, which is a pickup coil. Thus, based on the principle of the Villari effect, it is possible to calculate the stress applied to the base metal 370 based on the value of the alternating voltage. Then, the straining degree of the strain generating bodies 20 and 20A can be determined based on the calculated stress. When the detecting element 300 has the shape illustrated in FIG. 21 (a) and (b), the grid direction of the detecting element 300 is the same as the α-α' direction in FIG. 21 (a). By the principle described above, the detecting element 300 can detect a strain of the strain generating bodies 20 and 20A. That is, the detecting element 300 functions as a detecting element of a strain gauge.

It is preferable that the drive coil 320 is wound as uniformly as possible on the outer side of the sensing coil 380 and over the entirety of the region where the sensing coil 380 exists. This enables more uniform application of an alternating magnetic field to an entire region of the base metal 370 where the sensing coil 380 exists. As a result, it is possible to more minutely detect Villari effect-based changes in the magnetization magnitude of the base metal 370. Thus, the performance of the detecting element 300 is improved.

The insulating layer 360 may be formed only on a part, not the entirety, of the base metal 370. For example, parts of the base metal 370 over which the sensing coil 380 and the drive coil 320 are wound may be covered with the insulating layer 360, the insulating layer 360 may be covered with the insulating layer 350 including the sensing coil 380, and the insulating layer 350 may be covered with the insulating layer 340 including the drive coil 320.

When the base metal 370 is an approximately flat plate shape, the insulating layer 360 may be formed to surround the base metal 370 only in the coil winding direction. That is, in FIG. 21 (b), both ends of the base metal 370 in the y direction do not need to be covered with the insulating layer 360.

When the strain generating body 20 or 20A deforms (i.e., the strain generating body is strained) in the pulse wave sensor according to the present embodiment, the substrate 110 of the strain gauge (or the detecting element 300 itself) is strained. The detecting element 300 can detect a magnetic change due to this strain by the principle of the Villari effect described above.

The strain gauge including the detecting element 300 according to the present embodiment can be provided on the strain generating bodies 20 and 20A by any positioning patterns specified in the first embodiment and the modified examples of the first embodiment. That is, by using the detecting element 300 according to the present embodiment, it is possible to detect a strain of the strain generating bodies 20 and 20A as in the case of using an electric resistance-based strain gauge. Accordingly, the strain gauge according to the present embodiment can achieve the same effect as that achieved by the strain gauges 100 according to the first embodiment and the modified examples of the first embodiment.

### <Third embodiment>

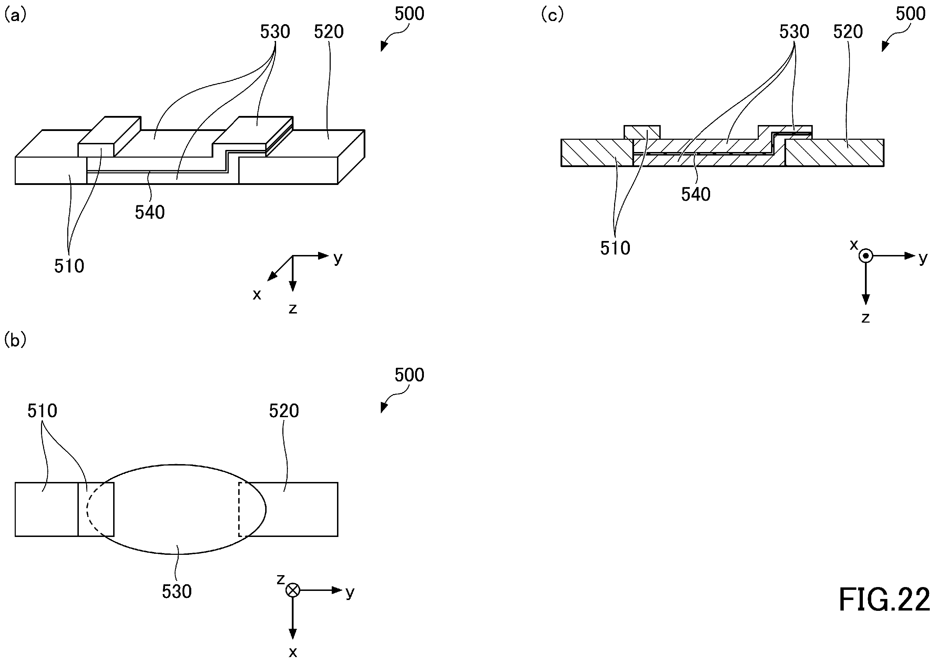

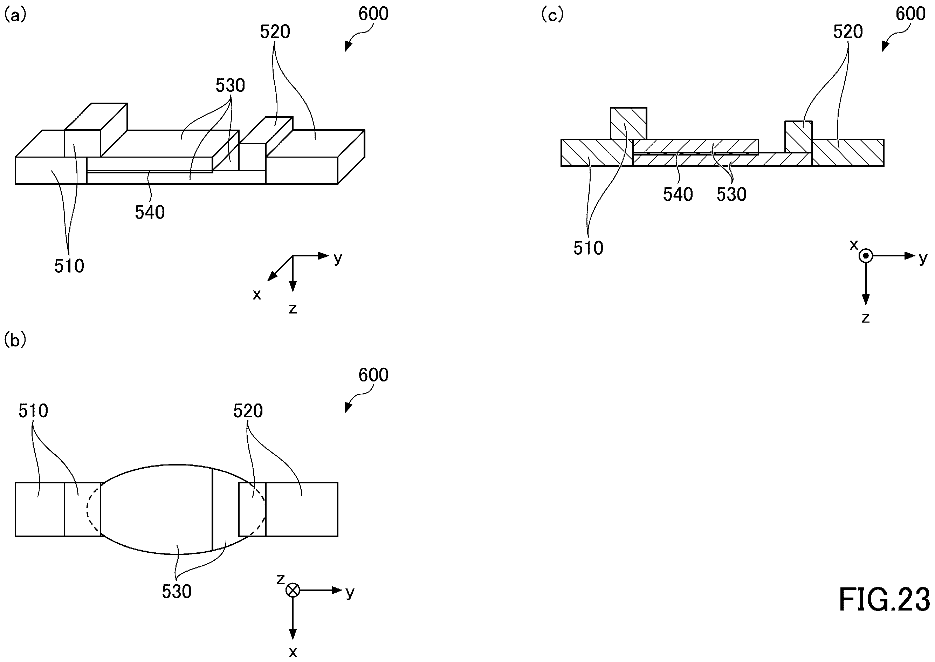

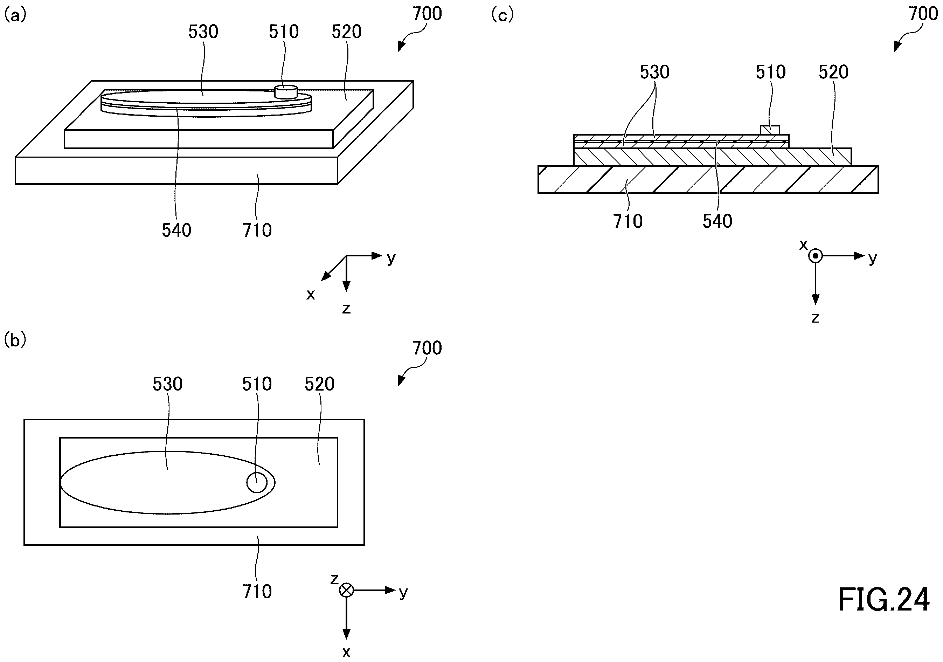

FIG. 22 is a view illustrating a detecting element 500, which is an example of a detecting element included in a strain gauge according to a third embodiment. FIG. 23 is a view illustrating a detecting element 600, which is another example of the detecting element according to the third embodiment. FIG. 24 is a view illustrating a detecting element 700, which is yet another example of the detecting element according to the third embodiment. FIGS. 22 to 24 (a) are perspective views of the detecting elements 500, 600, and 700, respectively. FIGS. 22 to 24 (b) are plan views of the detecting elements 500, 600, and 700, viewed from the z-axis negative direction side in the positive direction, respectively. FIGS. 22 to 24 (c) are cross-sectional views of the detecting elements 500, 600, and 700 along a surface parallel with the zy plane. None of the views of FIGS. 22 to 24 illustrate wirings extending from the detecting element. However, a wiring for connecting an upstream electrode 510 described below and a power source with each other, and a wiring for connecting a downstream electrode 520 and a power source with each other may be connected to the detecting elements 500, 600, and 700.

As illustrated in FIGS. 22 to 24 (a), the detecting elements 500, 600, and 700 each include an upstream electrode 510, a downstream electrode 520, magnetic films 530, and an insulating film 540. The insulating film 540 is sandwiched between the magnetic films 530 as illustrated. The magnetic films 530 and the insulating film 540 form a magnetic tunnel junction. That is, the detecting elements 500, 600, and 700 are each a structure obtained by connecting electrodes to a magnetic tunnel junction structure.

The upper surface of the detecting elements 500, 600, and 700 may be pasted on a substrate similar to the substrate 110 according to the second embodiment. The detecting element 500 may be pasted on the strain generating body 20 or 20A via the substrate. The detecting elements 500, 600, and 700 may each be a detecting element having a flat plate shape or a thin film shape as a whole. The detecting elements 500, 600, and 700 having a flat plate shape or a thin film shape are easier to paste on the substrate or on the strain generating body 20 or 20A. In addition, for example, the detecting elements 500, 600, and 700 may be used with their upper surface directly pasted on the strain generating body 20 or 20A.

The magnetic films 530 are magnetic nano-thin films. The insulating film 540 is an insulating nano-thin film. The materials of the magnetic films 530 and the insulating film 540 are not particularly limited so long as a magnetic tunnel junction structure can be formed. For example, cobalt-iron-boron, or 3d transition metal ferromagnetic materials such as Fe, Co, Ni, and the like, or alloys or the like containing them may be used as the magnetic films 530. Silicon oxide, silicon nitride, aluminum oxide, magnesium oxide, and the like may be used as the insulating film 540.

The upstream electrode 510 and the downstream electrode 520 are electrodes configured to apply a voltage across the magnetic tunnel junction structure. In the examples of FIGS. 22 to 24, the current flows from the upstream electrode 510 to the downstream electrode 520. In the case of, for example, FIG. 22 (c), when a voltage is applied across the upstream electrode 510 and the downstream electrode 520, electrons flow from the magnetic film 530 on the lower side (on the z-axis negative direction side) to the magnetic film 530 on the upper side (on the z-axis positive direction side) beyond the insulating film 540. This is a phenomenon referred to as the "tunnel effect", and the electric resistance against flowing of electrons via the insulating film 540 is referred to as the "tunnel resistance". In the examples of FIGS. 22 to 24, end portions of the junctions between various parts of the electrodes are processed such that no current shorting the magnetic tunnel junction structure will flow.

When the detecting element 500 is strained via the substrate 110 or the like, a magnetic change occurs in the tunnel junction structure. More specifically, the upper and lower magnetic films 530 are magnetized in different directions. When the upper and lower magnetic films 530 are magnetized in different directions in this way, the tunnel resistance is greater than when the directions in which they are magnetized are parallel (tunnel magnetic resistance effect). Thus, in the detecting element 500 having the configuration described above, the current flowing between the electrodes becomes smaller in accordance with the magnitude of the strain of the detecting element 500 (more strictly, the magnetic tunnel junction part). That is, the greater the strain, the higher the electric resistance. In this way, the detecting element 500 can detect a strain based on the current value relative to the applied voltage. Thus, by pasting the detecting element 500 on the strain generating body 20 or 20A, it is possible to measure a strain applied to the strain generating body 20 or 20A.

The detecting element having the magnetic tunnel junction structure is not limited to the example illustrated in FIG. 22. For example, it is possible to employ the detecting elements 600 and 700 illustrated in FIG. 23 and FIG. 24. The detecting element 600 illustrated in FIG. 23 and the detecting element 700 illustrated in FIG. 24 are the same as the detecting element 500 in that they are formed of the upstream electrode 510, the downstream electrode 520, the magnetic films 530, and the insulating film 540, and in the principle of detecting a strain by these components. The basic operations of the detecting elements 600 and 700 are also the same as that of the detecting element 500. The grid direction of the detecting elements 500, 600, and 700 corresponds to the y-axis direction (the y-axis positive direction and the y-axis negative direction) in FIG. 22 to FIG. 24. The detecting element 600 illustrated in FIG. 23 has a structure in which the upper magnetic film 530 and the lower magnetic film 530 are partially continuous, as illustrated. That is, the magnetic tunnel junction structure is formed only in a partial region of the magnetic films 530, and the tunnel magnetic resistance effect occurs in this structure. On the other hand, the detecting element 700 illustrated in FIG. 24 is pasted on the substrate 110 via a substrate 710. As illustrated in FIG. 22 to FIG. 24, the designs of the detecting elements may be appropriately changed in accordance with requirements such as size, durability, detecting target stress magnitude, and the like, as long as such design changes are not beyond the principle described above.

Basically, the strain generating bodies 20 and 20A according to the present embodiment may be of the same configuration and the same material as those of the strain generating bodies 20 and 20A according to the first embodiment. However, it is desirable that the strain generating bodies 20 and 20A are made of a nonmagnetic body. The strain generating bodies 20 and 20A according to the present embodiment may be manufactured using, for example, a nonmagnetic stainless steel as the material. The detecting elements 500, 600, and 700 may each be an approximately flat plate shape such as a film shape as a whole element. Thus, the detecting element 500 can be easily pasted on the strain generating bodies 20 and 20A. The detecting elements 500, 600, and 700 may have a structure, such as a drive coil structure or the like, for applying a weak magnetic field to the magnetic tunnel junction structure part. By applying a magnetic field to the magnetic tunnel junction structure part, it is possible to measure the tunnel magnetic resistance effect described above more stably, making it possible to detect a strain stably.

The "upstream electrode" and the "downstream electrode" of the detecting elements 500, 600, and 700 are the names used for convenience, and the current flowing direction may be reversed. That is, the current may be designed to flow from the downstream electrode 520 to the upstream electrode 510 in the detecting elements 500, 600, and 700 illustrated in FIG. 22 to FIG. 24.

In the pulse wave sensor according to the present embodiment, when the strain generating body 20 or 20A deforms (i.e., when the strain generating body is strained), the substrate of the strain gauge (or the detecting element 500, 600, or 700 itself) is strained. The detecting element 500, 600, or 700 can detect a magnetic change caused due to this strain based on the principle of the tunnel magnetic resistance effect described above.

The strain gauges including the detecting elements 500, 600, and 700 according to the present embodiment can be provided on the strain generating bodies 20 and 20A at any positions specified in the first embodiment and the modified examples of the first embodiment. That is, by using the detecting elements 500, 600, and 700 according to the present embodiment, it is possible to detect a strain of the strain generating bodies 20 and 20A, as in the case of using an electric resistance-based strain gauge. Accordingly, the strain gauge according to the present embodiment can achieve the same effect as that achieved by the strain gauges 100 according to the first embodiment and the modified examples of the first embodiment.

### <Fourth embodiment>

The detector according to the present disclosure may be a semiconductor strain gauge, a capacitive pressure sensor, or an optical fiber strain gauge. The detector according to the present disclosure may be a mechanical pressure sensor, a vibrating pressure sensor, or a piezoelectric pressure sensor. Principles of various strain gauges and pressure sensors will be described below.

### (Semiconductor strain gauge)

A semiconductor strain gauge is a strain gauge configured to detect a strain by utilizing the piezoresistive effect of a semiconductor. That is, a semiconductor strain gauge is a strain gauge using a semiconductor as a strain detecting element.

It is known that when a stress is applied to a semiconductor, the crystal lattice of the semiconductor strains and the number and mobility of carriers in the semiconductor change, resulting in a change in the electric resistance. The semiconductor strain gauge can be used while being directly pasted on the strain generating body 20 or 20A like the electric resistance-based metallic strain gauge. In this case, when the strain generating body 20 or 20A expands or contracts, the pasted semiconductor (more specifically, the crystal lattice of the semiconductor) strains, to change the electric resistance. Thus, by measuring the electric resistance, it is possible to determine the amount of the strain of the strain generating body 20 or 20A.

Moreover, the semiconductor strain gauge can be configured as a strain sensor having a diaphragm structure. In this case, for example, the strain sensor includes a nonmetallic diaphragm (or a product including an electrically insulating layer on a metallic diaphragm), and a semiconductor (for example, a semiconductor made of a silicon thin film) formed on the diaphragm. In such a structure including a diaphragm, when the diaphragm strains due to a perpendicular stress applied to the diaphragm, the electric resistance of the semiconductor changes. Thus, by measuring the electric resistance, it is possible to determine the amount of the strain of the diaphragm (and, accordingly, the amount of the strain of the strain generating body 20 or 20A).

### (Capacitive pressure sensor)

A capacitive pressure sensor is a pressure sensor configured to measure a pressure applied to a diaphragm in the form of a change in the capacitance across a pair of electrodes. That is, the capacitive pressure sensor is a pressure sensor using a pair of electrodes as detecting elements. For example, the capacitive pressure sensor includes a diaphragm as a movable electrode, and one or more fixed electrodes. For example, the diaphragm is made of silicon containing an impurity (i.e., silicon functioning as a conductor) or the like.

When a pressure is applied to the diaphragm, the diaphragm is displaced, to change the distance between the fixed electrodes and the movable electrode. It is known that the capacitance across the electrodes is determined in accordance with the distance between the electrodes, provided that the permittivity of the medium between the electrodes and the electrode areas are constant. Thus, by measuring the capacitance, it is possible to determine the amount of the displacement of the diaphragm (i.e., the magnitude of the pressure).

### (Optical fiber strain gauge)

An optical fiber strain gauge is a strain gauge configured to detect a strain by using an optical fiber including Fiber Bragg Gratings (FBGs). That is, an optical fiber strain gauge is a strain gauge using an optical fiber as a strain detecting element. The FBGs are diffraction gratings configured to reflect light in a different manner from the other parts of the optical fiber. The individual gratings are formed at constant intervals. When the optical fiber strains and becomes extended, the grating interval of the FBGs is increased. Therefore, a wavelength change occurs between incident light (e.g., laser light) coming into the optical fiber and its reflected light. Moreover, when the optical fiber strains and becomes contracted, the grating interval of the FBGs is reduced. Therefore, a wavelength change occurs between incident light (e.g., laser light) coming into the fiber and its reflected light.

By pasting the optical fiber having this property on the strain generating body 20 or 20A and measuring the wavelength spectrum of reflected light in the optical fiber, it is possible to determine the amount of the strain of the optical fiber (i.e., the amount of the strain of the strain generating body 20 or 20A). The optical fiber strain gauge may be a strain gauge configured to determine the amount of a strain of the optical fiber based on a change in the frequency of Brillouin-scattered light occurring in the optical fiber.

### (Mechanical pressure sensor)

A mechanical pressure sensor is a sensor configured to determine a pressure applied to a mechanical structure by measuring the amount of displacement of the structure. For example, the mechanical pressure sensor includes a spring or a bent tube, and is configured to measure the amount of expansion or contraction of the spring or the amount of expansion or contraction of the bent tube. The amount of their expansion or contraction (i.e., the amount of displacement) changes in accordance with the magnitude of the pressure applied to the spring or the bent tube. Thus, by measuring the amount of expansion or contraction, it is possible to determine the pressure applied to the spring or the bent tube. The shape and the size of the spring or the bent tube may be appropriately determined in accordance with the size and the shape of the target to which the mechanical pressure sensor is attached.

### (Vibrating pressure sensor)

A vibrating pressure sensor is a sensor configured to detect a pressure by utilizing a phenomenon that the natural frequency of an elastic beam changes in accordance with a pressure (an axial load) that occurs along the axis of the elastic beam. The vibrating pressure sensor can be used while being directly pasted on the strain generating bodies 20 and 20A, like the electric resistance-based metallic strain gauge. Moreover, for example, the vibrating pressure sensor may be a pressure sensor including a diaphragm formed on a substrate and a beam-like vibrator formed on the surface of the diaphragm.

In any case, when the strain generating body 20 or 20A strains, the straining pressure is directly or indirectly transmitted to the vibrator, to apply an axial load on the vibrator. The natural frequency of the vibrator changes in accordance with the axial load. Thus, by measuring the natural frequency of the vibrator, it is possible to determine the magnitude of the pressure on the strain generating body 20 or 20A.

### (Piezoelectric pressure sensor)

A piezoelectric pressure sensor is a sensor including a piezoelectric element (also referred to as a piezo element), and configured to detect a pressure by utilizing the characteristics of the piezoelectric element. The piezoelectric element has a characteristic of generating an electromotive force corresponding to a force applied thereto, when it deforms (strains) in response to the application of the force. The piezoelectric element also has a characteristic of generating a force corresponding to a voltage applied thereto and undergoing expansion or contraction, in response to the application of the voltage.

The piezoelectric pressure sensor can determine the force applied to the piezoelectric element (i.e., the amount of a strain of the piezoelectric element) by measuring the electromotive force of the piezoelectric element. Thus, by pasting the piezoelectric pressure sensor on the strain generating body 20 or 20A, it is possible to determine the amount of a strain of the strain generating body 20 or 20A.

As described above, also in a case of using the semiconductor, capacitive, optical fiber, vibrating, mechanical, and piezoelectric pressure sensors, it is possible to achieve the same effect as that achieved by the strain gauge 100 according to the first embodiment and the modified examples of the first embodiment.

Preferred embodiments and the like have been described above. However, the pulse wave sensor according to the present disclosure is not limited to the embodiments and modified examples described above. For example, various modifications and substitutions are applicable to the pulse wave sensor according to the embodiments and the like described above, without departing from the scope specified in the claims.

The present international application claims priority to Japanese Patent Application No. 2022-033658 filed March 4, 2022 and Japanese Patent Application No. 2023-014131 filed February 1, 2023. The entire contents of Japanese Patent Application No. 2022-033658 and Japanese Patent Application No. 2023-014131 are incorporated herein by reference.

### REFERENCE SIGNS LIST

1,1A,1B,1C: pulse wave sensor, 10: housing, 20,20A: strain generating body, 20m: upper surface, 20n: lower surface, 21: base portion, 22: beam portion, 23,53: load portion, 24: extension portion, 30: wire rod, 50,50A: resin layer, 100: strain gauge, 110: substrate, 110a: upper surface, 130: resistor, 140: wiring, 150: electrode, 160: cover layer, 130e₁, 130e₂: end, 300,500,600,700: detecting element, 310: base layer, 320: drive coil, 340,350,360: insulating layer, 370: base metal, 380: sensing coil, 510: upstream electrode, 520: downstream electrode, 530: magnetic film, 540: insulating film, 710: substrate

## Claims

1. A pulse wave sensor, comprising:
a strain generating body with a circular opening;
a resin layer covering one surface of the strain generating body; and
a strain gauge provided on the other surface of the strain generating body positioned on a side opposite to the one surface, the strain gauge including a Cr mixed phase film as a resistor,
wherein where a diameter of the circular opening is d [mm] and a thickness of the strain generating body is t [mm],
when a material of the strain generating body is SUS and d is 32, a required range of t is 0.059≤t≤0.124,
when the material is SUS and d is 22, a required range of t is 0.046≤t≤0.099,
when the material is SUS and d is 13, a required range of t is 0.030≤t≤0.067,
when the material is SUS and d is 7, a required range of t is 0.026≤t≤0.034,
when the material is copper and d is 32, a required range of t is 0.084≤t≤0.166,
when the material is copper and d is 22, a required range of t is 0.066≤t≤0.132,
when the material is copper and d is 13, a required range of t is 0.044≤t≤0.088,
when the material is copper and d is 7, a required range of t is 0.032≤t≤0.050,
when the material is aluminum and d is 32, a required range of t is 0.097≤t≤0.212,
when the material is aluminum and d is 22, a required range of t is 0.079≤t≤0.168,
when the material is aluminum and d is 13, a required range of t is 0.050<t<0.107,
when the material is aluminum and d is 7, a required range of t is 0.038<t<0.063, and
the pulse wave sensor is configured to detect a pulse wave based on a change in a resistance value of the resistor in response to a deformation of the strain generating body.

2. A pulse wave sensor, comprising:
a strain generating body;
a resin layer covering one surface of the strain generating body; and
a strain gauge provided on the other surface of the strain generating body positioned on a side opposite to the one surface, the strain gauge including a Cr mixed phase film as a resistor,
wherein the strain generating body includes:
a base portion with a circular opening;
a beam portion bridging an inside of the base portion; and
a load portion provided on the beam portion,
the strain generating body has a flat plate shape, and
the pulse wave sensor is configured to detect a pulse wave based on a change in a resistance value of the resistor in response to a deformation of the strain generating body.

3. A pulse wave sensor, comprising:
a strain generating body made of a nonmetallic material; and
a strain gauge provided on the strain generating body and including a Cr mixed phase film as a resistor,
wherein the strain generating body includes:
a base portion with a circular opening;
a beam portion bridging an inside of the base portion; and
a load portion provided on the beam portion,
the strain generating body has a flat plate shape, and
the pulse wave sensor is configured to detect a pulse wave based on a change in a resistance value of the resistor in response to a deformation of the strain generating body.

4. The pulse wave sensor according to claim 3, further comprising:
a resin layer covering one surface of the strain generating body,
wherein the strain gauge is provided on the other surface of the strain generating body positioned on a side opposite to the one surface.

5. The pulse wave sensor according to claim 1,
wherein the strain generating body includes:
a base portion with a circular opening;
a beam portion bridging an inside of the base portion; and
a load portion provided on the beam portion.

6. The pulse wave sensor according to claim 5,
wherein the strain generating body has a flat plate shape.

7. The pulse wave sensor according to any one of claims 2 to 6,
wherein the beam portion has two beams that cross each other in a cross shape in a plan view,
a region where the two beams cross includes a center of the circular opening, and
the load portion is provided on the region where the two beams cross.

8. The pulse wave sensor according to claim 7,
wherein the pulse wave sensor includes four strain gauges, each of which is the strain gauge,
two of the four strain gauges are positioned on the beam having its longitudinal direction in a first direction, at a portion of the beam close to the load portion, so that the two of the four strain gauges face each other across the load portion in the plan view, and
the other two of the four strain gauges are positioned on the beam having its longitudinal direction in a second direction orthogonal to the first direction, at a portion of the beam close to the base portion, so that the other two of the four strain gauges face each other across the load portion in the plan view.

9. The pulse wave sensor according to claim 7 or 8,
wherein a length of each of the beams is approximately equal to a diameter of the circular opening.

10. The pulse wave sensor according to any one of claims 7 to 9,
wherein in each of the beams, a width other than the region where the two beams cross is constant.

11. The pulse wave sensor according to any one of claims 1 to 10, further comprising:
a second resin layer provided on an other surface of the strain generating body and covering the strain gauge.

12. A pulse wave sensor, comprising:
a strain generating body with a circular opening;
a resin layer covering one surface of the strain generating body; and
a detector provided on the other surface of the strain generating body positioned on a side opposite to the one surface,
wherein where a diameter of the circular opening is d [mm] and a thickness of the strain generating body is t [mm],
when a material of the strain generating body is SUS and d is 32, a required range of t is 0.059≤t≤0.124,
when the material is SUS and d is 22, a required range of t is 0.046≤t≤0.099,
when the material is SUS and d is 13, a required range of t is 0.030≤t≤0.067,
when the material is SUS and d is 7, a required range of t is 0.026≤t≤0.034,
when the material is copper and d is 32, a required range of t is 0.084≤t≤0.166,
when the material is copper and d is 22, a required range of t is 0.066≤t≤0.132,
when the material is copper and d is 13, a required range of t is 0.044≤t≤0.088,
when the material is copper and d is 7, a required range of t is 0.032≤t≤0.050,
when the material is aluminum and d is 32, a required range of t is 0.097≤t≤0.212,
when the material is aluminum and d is 22, a required range of t is 0.079≤t≤0.168,
when the material is aluminum and d is 13, a required range of t is 0.050<t<0.107,
when the material is aluminum and d is 7, a required range of t is 0.038<t<0.063,
the detector is configured to detect either or both of a deformation of the strain generating body and a pressure applied to the strain generating body, and
the pulse wave sensor is configured to detect a pulse wave based on a change in either or both of the deformation and the pressure detected by the detector.

13. A pulse wave sensor, comprising:
a strain generating body;
a resin layer covering one surface of the strain generating body; and
a detector provided on the other surface of the strain generating body positioned on a side opposite to the one surface,
wherein the strain generating body includes:
a base portion with a circular opening;
a beam portion bridging an inside of the base portion; and
a load portion provided on the beam portion,
the strain generating body has a flat plate shape,
the detector is configured to detect either or both of a deformation of the strain generating body and a pressure applied to the strain generating body, and
the pulse wave sensor is configured to detect a pulse wave based on a change in either or both of the deformation and the pressure detected by the detector.

14. A pulse wave sensor, comprising:
a strain generating body made of a nonmetallic material; and
a detector provided on the strain generating body,
wherein the strain generating body includes:
a base portion with a circular opening;
a beam portion bridging an inside of the base portion; and
a load portion provided on the beam portion,
the strain generating body has a flat plate shape,
the detector is configured to detect either or both of a deformation of the strain generating body and a pressure applied to the strain generating body, and
the pulse wave sensor is configured to detect a pulse wave based on a change in either or both of the deformation and the pressure detected by the detector.

15. The pulse wave sensor according to claim 14, further comprising:
a resin layer covering one surface of the strain generating body,
wherein the detector is provided on the other surface of the strain generating body positioned on a side opposite to the one surface.

16. The pulse wave sensor according to claim 12,
wherein the strain generating body includes:
a base portion with a circular opening;
a beam portion bridging an inside of the base portion; and
a load portion provided on the beam portion.

17. The pulse wave sensor according to claim 16,
wherein the strain generating body has a flat plate shape.

18. The pulse wave sensor according to any one of claims 13 to 17,
wherein the beam portion has two beams that cross each other in a cross shape in a plan view,
a region where the two beams cross includes a center of the circular opening, and
the load portion is provided on the region where the two beams cross.

19. The pulse wave sensor according to claim 18,
wherein the pulse wave sensor includes four detectors, each of which is the detector,
two of the four detectors are positioned on the beam having its longitudinal direction in a first direction, at a portion of the beam close to the load portion, so that the two of the four detectors face each other across the load portion in the plan view, and
the other two of the four detectors are positioned on the beam having its longitudinal direction in a second direction orthogonal to the first direction, at a portion of the beam close to the base portion, so that the other two of the four detectors face each other across the load portion in the plan view.

20. The pulse wave sensor according to claim 18 or 19,
wherein a length of each of the beams is approximately equal to a diameter of the circular opening.

21. The pulse wave sensor according to any one of claims 18 to 20,
wherein in each of the beams, a width other than the region where the two beams cross is constant.

22. The pulse wave sensor according to any one of claims 12 to 21, further comprising:
a second resin layer provided on an other surface of the strain generating body and covering the detector.

23. The pulse wave sensor according to any one of claims 12 to 22,
wherein the detector includes a detecting element configured to detect a magnetic change caused due to the deformation of the strain generating body.

24. The pulse wave sensor according to claim 23,
wherein the detecting element includes a magnetic body, and
the detecting element is a detecting element configured to detect a change in a magnetization magnitude of the magnetic body in response to a pressure being applied to the magnetic body due to the deformation of the strain generating body.

25. The pulse wave sensor according to claim 23,
wherein the detecting element includes a structure of a magnetic tunnel junction in which an insulating film is sandwiched between magnetic films, and
the detecting element is a detecting element configured to detect the magnetic change caused in the structure due to the deformation of the strain generating body.

26. The pulse wave sensor according to any one of claims 12 to 22,
wherein the detector is a semiconductor strain gauge.

27. The pulse wave sensor according to any one of claims 12 to 22,
wherein the detector is a capacitive pressure sensor.

28. The pulse wave sensor according to any one of claims 12 to 22,
wherein the detector is an optical fiber strain gauge.
